# EUROPEAN PATENT APPLICATION

(11) **EP 4 790 634 A1**
(43) Date of publication of application: **12.08.2026**
(21) Application number: 26154415.9
(22) Date of filing: 27.01.2026
(51) Int. Cl.: G06T 3/40, G06T 12/00, G06T 12/20

(54) **MULTIRESOLUTION IMAGE RECONSTRUCTION WITH UPSCALING**

(30) Priority: 07.02.2025 US 202519047654
(71) Applicant: Siemens Healthineers International AG, 6312 Steinhausen (CH)
(72) Inventor: STRZELECKI, Adam, 5405 Dattwil (CH); HOFMANN, Urs, 5405 Dattwil (CH); THOMPSON, Stephen, Palo Alto, 94304-1038 (US)
(74) Representative: Mathisen & Macara LLP

(57) **Abstract**

Example methods (1200) and systems for multiresolution image reconstruction with upscaling are described. In one example, a computer system may obtain first volume image data (1210) associated with a first resolution level. The computer system may upscale (1220) the first volume image data associated with the first resolution level to generate second volume image data (1230) associated with a second resolution level, which is higher than the first resolution level. The computer system may obtain (1240) mask image data (1250) that identifies a region of interest (ROI) associated with a target structure. Based on the second volume image data and the mask image data, the computer system may generate multiresolution volume image data (1270) that includes at least a first voxel associated with the second resolution level outside of the ROI, and a second voxel associated with a third resolution level within the ROI.

## Description

### CROSS-REFERENCE TO RELATED APPLICATIONS

The present application (Attorney Docket No. 09832) is related in subject matter to EP Patent Application entitled "Iterative Image Reconstruction with Upscaling" (Attorney Docket No. 09831, claiming priority from 19/047,652) filed on the same day, and United States Patent Application No. 19/006,121 (corresponding to EP25223983.5, Attorney Docket No. 09774, entitled "Image Reconstruction Using Multiple Reconstruction Chains for Radiation Therapy"), filed on December 30, 2024, which are incorporated herein by reference.

### BACKGROUND

Radiation therapy is a widely used cancer treatment modality that uses high-energy radiation to reduce or eliminate cancerous tumors. In practice, applied radiation does not inherently discriminate between a tumor and proximal healthy structures, such as organs, healthy tissues, etc. Ideally, the objective is to deliver a lethal or curative radiation dose to the tumor, while maintaining an acceptable dose level in the healthy structures. Image reconstruction may be performed to generate volume image data based on projection image data associated with a patient. Based on the volume image data, clinicians and planning tools may more accurately target tumors while sparing healthy structures from unnecessary radiation exposure. It is therefore desirable to improve the quality of image reconstruction to enhance the efficacy of radiation therapy and ultimately improve patient outcomes.

### BRIEF DESCRIPTION OF DRAWINGS

FIG. 1 is a flowchart illustrating an example process for a computer system to perform iterative image reconstruction with upscaling for radiation therapy;
FIG. 2A is a schematic diagram illustrating conventional iterative image reconstruction;
FIG. 2B is a schematic diagram illustrating a first example of iterative image reconstruction with one upscaling stage;
FIG. 3 is a schematic diagram illustrating a second example of iterative image reconstruction with multiple upscaling stages;
FIG. 4 is a schematic diagram illustrating an example radiation therapy system that includes a computer system to perform image reconstruction during a pre-treatment phase of radiation therapy;
FIG. 5 is a schematic diagram illustrating an example radiation therapy system that includes a computer system to perform image reconstruction during a treatment phase of radiation therapy;
FIG. 6 is a flowchart of an example detailed process for a computer system to perform iterative image reconstruction with upscaling for radiation therapy;
FIG. 7 is a flowchart of an example process for a computer system to implement a resolution refinement stage in the example in FIG. 6;
FIG. 8 is a flowchart of an example process for a computer system to implement an upscaling stage in the example in FIG. 6;
FIG. 9 is a flowchart of an example process for a computer system to perform iterative image reconstruction using an algebraic reconstruction technique (ART) and/or a penalized likelihood (PL) technique;
FIG. 10A is a schematic diagram illustrating a first example user interface (UI) view for a user to interact with first volume image data;
FIG. 10B is a schematic diagram illustrating a second example UI view for a user to interact with output volume image data;
FIG. 11 is a schematic diagram illustrating multiple UI views for displaying multiple sets of volume image data;
FIG. 12 is a flowchart illustrating an example process for a computer system to perform multiresolution image reconstruction with upscaling for radiation therapy;
FIG. 13 is a schematic diagram illustrating a first example of multiresolution image reconstruction with one upscaling stage;
FIG. 14 is a schematic diagram illustrating a second example of multiresolution image reconstruction with multiple upscaling stages; and
FIG. 15 is a flowchart of an example detailed process for a computer system to perform multiresolution image reconstruction with upscaling for radiation therapy.

### SUMMARY

In accordance with a first facet of the invention, there is provided a method for a computer system to perform multiresolution image reconstruction with upscaling, the method as defined by claim 1.

In accordance with a second facet of the invention, there is provided a non-transitory computer-readable medium, as defined by claim 8.

In accordance with a third facet of the invention, there is provided an imaging system as defined by claim15.

Optional features are defined by the dependent claims.

The term "upscaling" or "upscale" may refer generally to transforming lower-resolution volume image data into higher-resolution volume image data, thereby increasing its resolution level.

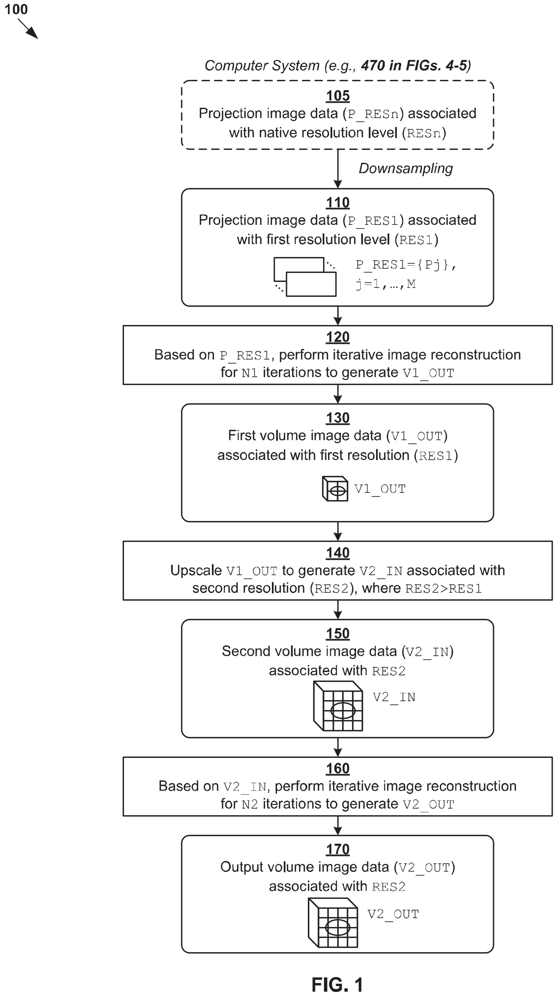

Examples of the present disclosure may be implemented to improve the computational efficiency of image reconstruction for radiation therapy, leading to faster clinical workflows and improved patient outcomes. According to a first aspect of the present disclosure, methods and systems for iterative image reconstruction with upscaling are described. In one example, a computer system may obtain projection image data associated with a target structure of a patient. The computer system may perform iterative image reconstruction for a first number of iterations to generate first volume image data based on the projection image data. The first volume image data may be associated with a first resolution level. The computer system may upscale the first volume image data associated with the first resolution level to generate second volume image data associated with a second resolution level, which is higher than the first resolution level. The computer system may perform iterative image reconstruction for a second number of iterations to generate output volume image data based on the second volume image data. The output volume image data may be associated with the second resolution level. Various embodiments will be explained using FIGs. 1, 2B-11.
Earlier iterations of the iterative image reconstruction may recover low-frequency features first, whereas later iterations may recover higher-frequency features.

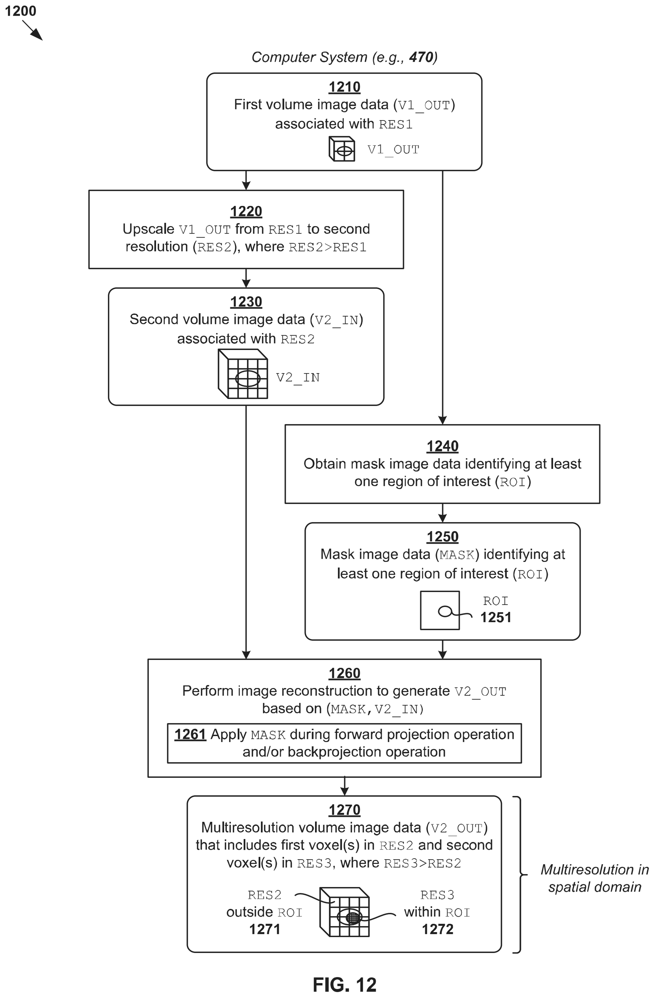

According to a second aspect of the present disclosure, methods and systems for multiresolution image reconstruction with upscaling are described. In one example, a computer system may obtain first volume image data that is generated based on projection image data associated with a target structure of a patient. The computer system may upscale the first volume image data associated with a first resolution level to generate second volume image data associated with a second resolution level, which is higher than the first resolution level. The computer system may obtain mask data that identifies a region of interest (ROI) associated with the target structure. The computer system may generate multiresolution volume image data based on the second volume image data and the mask image data. The multiresolution volume image data may include at least a first voxel associated with the second resolution level outside of the ROI, and a second voxel associated with a third resolution level within the ROI. Various embodiments will be explained using FIGs. 12-15.

Examples of the present disclosure may further comprise a computer system that includes a processor and a non-transitory computer-readable medium having stored thereon instructions that, when executed by the processor, cause the processor to perform aspect(s) of the above method(s). Another aspect may include a non-transitory computer-readable storage medium that includes a set of instructions which, in response to execution by a processor or a computer, cause the processor or the computer to perform aspect(s) of the above method(s). Yet another aspect may include a computer program comprising instructions which, when executed by a processor or a computer, cause the processor or the computer to carry out aspect(s) of the above method(s). A further aspect may include an imaging system, which may include an imaging source and a detector (also known as an imager) to acquire projection image data; and a computer system to perform aspect(s) of the above method(s).

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here. It will be readily understood that the aspects of the present disclosure, as generally described herein, and illustrated in the drawings, can be arranged, substituted, combined, and designed in a wide variety of different configurations, all of which are explicitly contemplated herein. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

### Overview

Imaging modalities such as cone-beam computed tomography (CBCT) are widely used in clinical settings for diagnosis of various diseases, as a tool during surgical procedures, as a positioning tool prior to radiation therapy, etc. To facilitate visualization of a patient's internal anatomy, image reconstruction may be performed to generate three-dimensional (3D) volume image data of a patient's internal anatomy based on two-dimensional (2D) projection image data (also known as projections) that is acquired using an imaging system.

As used herein, the term "image reconstruction" may refer generally to a process for generating volume image data based on projection image data. The term "projection image data" (used interchangeably with "2D projection data," "2D projection image" and "projections") may refer generally to data representing properties of illuminating radiation rays transmitted through a subject. The term "volume image data" (also known as "reconstruction" or "reconstructed image") may refer generally to data representing a 3D reconstruction that is generated based on projection image data.

Current CBCT imaging systems may be configured to provide high-resolution axial slices, such as 512 x 512 pixels with ~0.5 mm/pixel or mm pixel size for head scans, up to ~1 mm/pixel or mm pixel size for body in-plane resolution, etc. However, these systems employ relatively coarse longitudinal (out-of-plane) sampling intervals, such as 2 mm/pixel or mm pixel size for image guided radiation therapy (IGRT) and 3 mm/pixel or mm pixel size for CBCT imaging for treatment planning (CBCTp). In general, the use of coarse longitudinal sampling is driven by the intention to reduce the number of slices as well as the computational time and effort required for analyzing and delineating visible anatomical structures. However, the use of coarse longitudinal sampling may lead to undesirable side effects.

First, CBCT image reconstruction using regular non-matching projectors may produce sampling artifacts, such as aliasing and shading caused by the Gibbs phenomenon when recovering high-frequency components, etc. Second, while axial views may retain usable details, sagittal and/or coronal views may be prone to severe sampling artifacts, compromising image quality in those planes. Third, the feasibility of further functionality improvement, such as arbitrary plane slicing, may become limited by the coarse sampling approach.

In principle, existing image reconstruction algorithms may generate images with much higher longitudinal resolution using the current generation of flat panel imagers. The use of cubic voxels (i.e., grid with the same resolution in every direction) offers several potential advantages but may require improvements for feasibility. In practice, the time required for image reconstruction depends on the voxel grid resolution. Higher-resolution grids may significantly increase the computational load and time needed for image reconstruction. It is desirable to implement higher-resolution image reconstruction in a more efficient manner.

### First aspect: iterative image reconstruction with upscaling

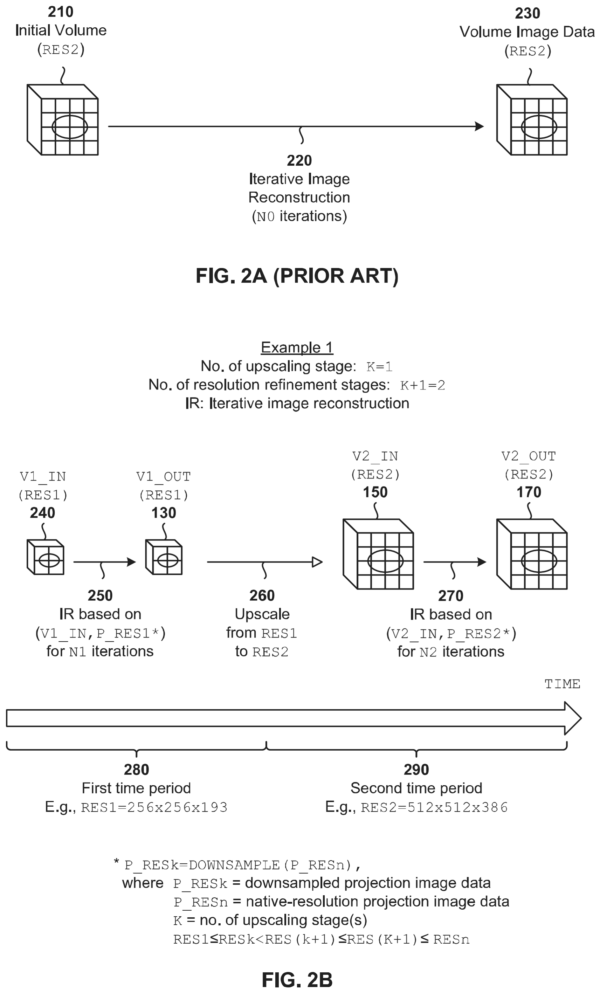

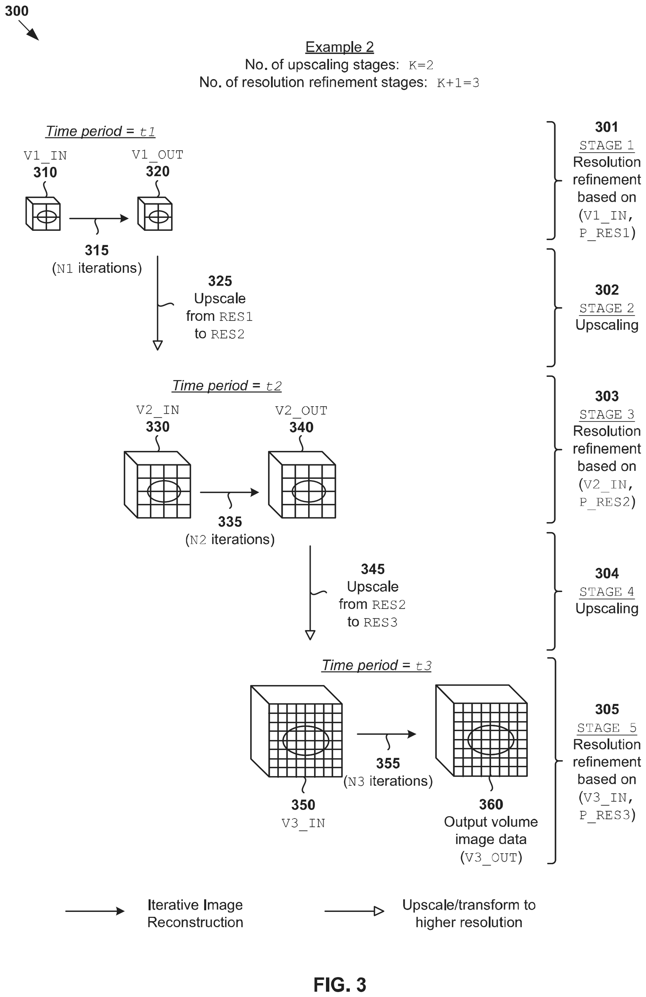

According to a first aspect of the present disclosure, methods and computer systems for iterative image reconstruction with upscaling are described. Using examples of the present disclosure, iterative image reconstruction may be implemented using multiple stages that include *K* upscaling stage(s) and *K +* 1 resolution refinement stages associated with respective *K* + 1 resolution levels. That is, the iterative image reconstruction may be implemented using multiple stages that include a number of upscaling stages and a number of resolution refinement stages, wherein the number of resolution refinement stages is one more than the number of upscaling stages. At least one upscaling stage (i.e., *K* ≥ 1) may be implemented. The case *of K =* 1 (i.e., one upscaling stage) will be explained using FIG. 1 and FIG. 2B. The case of *K* = 2 (i.e., two upscaling stages) will be explained using FIG. 3. Depending on the desired implementation, the highest resolution level (*K* + 1) may be configured to be lower than, or equal to, a native resolution level (denoted as RESn) associated with an imaging system.

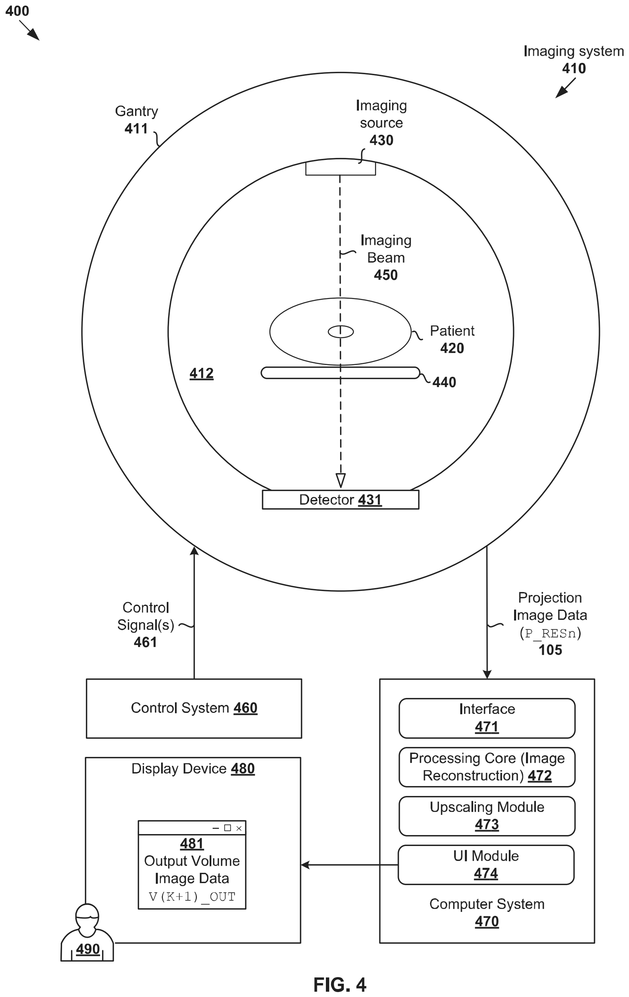

In more detail, FIG. 1 is a flowchart of an example process (see 100) for a computer system to perform iterative image reconstruction with upscaling for radiation therapy. Example process 100 may include one or more operations, functions, or actions illustrated by one or more blocks, such as 110 to 170. Depending on the desired implementation, various blocks may be combined into fewer blocks, divided into additional blocks, and/or eliminated. Any suitable computer system may be configured to perform various examples of the present disclosure, such as computer system 470 in FIGs. 4-5 (to be explained below).

At 110 in FIG. 1, computer system 470 in FIGs. 4-5 may obtain projection image data associated with a target structure of a patient requiring radiation therapy. Projection image data 110 (denoted as P_RES1) may be associated with a first resolution level (denoted as RES1). Projection image data 110 may include a set of multiple (*M*) projection images denoted as *Pⱼ*, where *j* = 1, ..., *M*. In practice, projection image data 110 (P_RES1) may represent first downsampled projection image data that is generated by downsampling native-resolution projection image data 105 (P_RESn). The native resolution level (RESn) may represent a resolution level associated with an imaging or image acquisition system. That is, the computer system may obtain projection image data by downsampling native resolution projection image data, the native resolution level representing a resolution level associated with an imaging or image acquisition system.

As used herein, the term "obtain" or "obtaining" may refer generally to receiving or retrieving data from any suitable source, such as an imaging system, a module/component of the same or a different computer system, or a datastore storing the data. The term "target structure" may refer generally to any suitable structure of interest, such as tumor, organ-at-risk (OAR), healthy tissue, bony structure (e.g., vertebra), etc. Note that projection image data 110 is not limited to being obtained from a single detector; it could originate from multiple sources, angles, energy levels, or even imaging modalities.

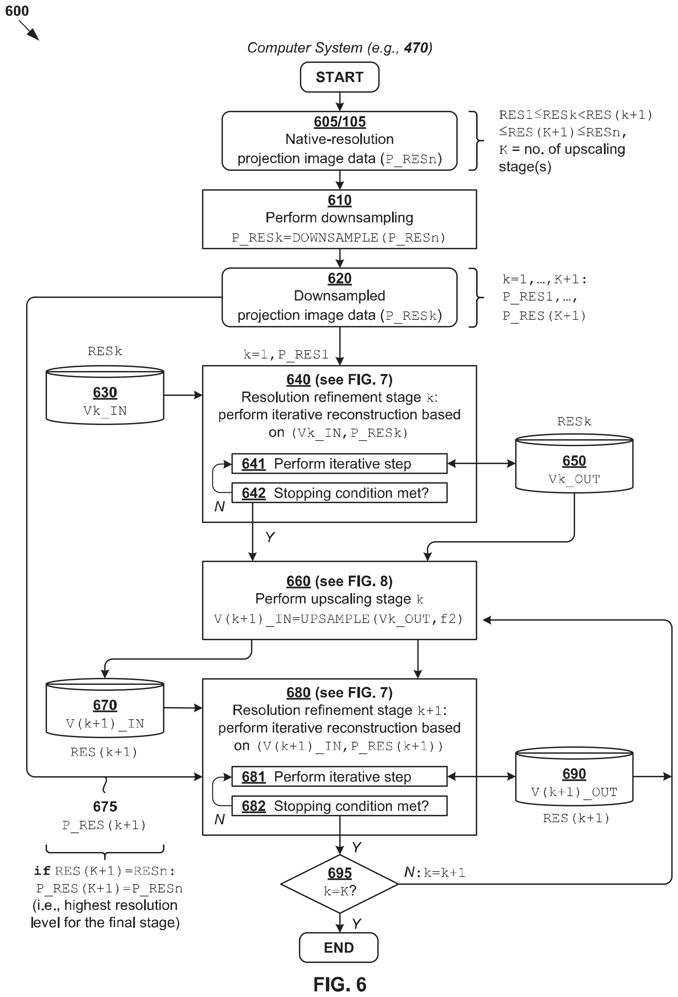

At 120 in FIG. 1, based on projection image data 110, computer system 470 may perform iterative image reconstruction for a first number of iterations (denoted as N1). Block 120 may be performed to generate first volume image data (denoted as V1_OUT 130) associated with RES1. As used herein, the term "iterative image reconstruction" or "iterative reconstruction" may refer generally to a computational process to generate volume image data based on projection image data through multiple iterations. Unlike conventional reconstruction approaches that apply a direct mathematical formula, iterative reconstruction may be performed iteratively to refine the volume image data, gradually improving its accuracy. An example algorithm for iterative image reconstruction will be described using FIGs. 6-7.

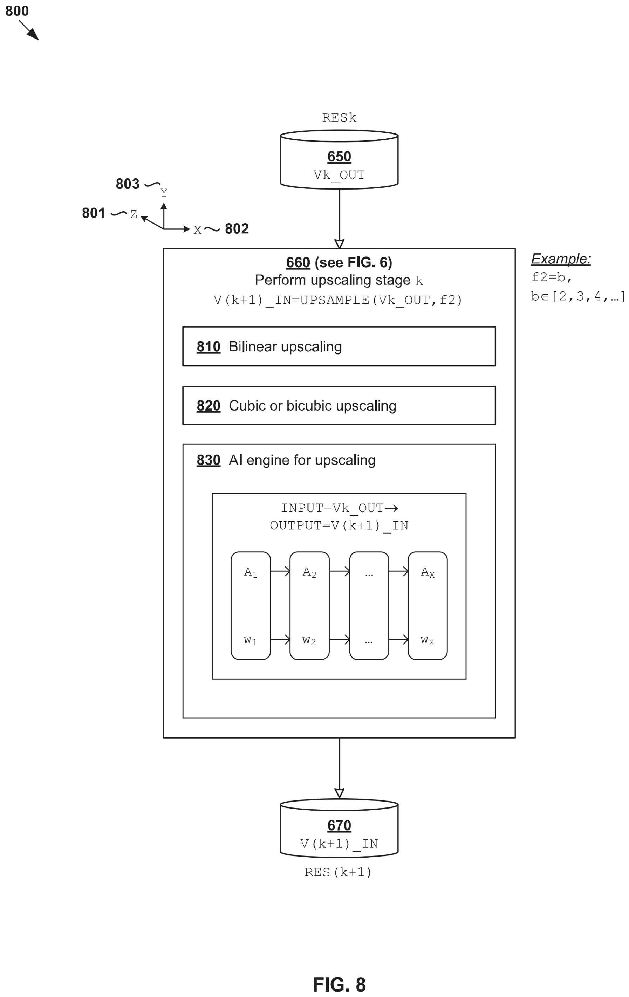

At 140 in FIG. 1, computer system 470 may upscale V1_OUT 130 associated with RES1 to generate second volume image data (denoted as V2_IN 150) associated with a second, higher resolution (denoted as RES2), i.e., RES2 > RES1. Throughout the present disclosure, the term "upscaling" or "upscale" may refer generally to transforming lower-resolution volume image data into higher-resolution volume image data, thereby increasing its resolution level. As will be described further using FIGs. 6 and 8, any suitable approach for upscaling may be implemented at block 125, such as bilinear upscaling, cubic upscaling, artificial intelligence (AI) engine for upscaling, etc. In general, more sophisticated approaches may be implemented to further reduce the number of higher-resolution iterations required to recover fine/high-frequency details.

At 160 in FIG. 1, based on V2_IN 150, computer system 470 may perform iterative image reconstruction for a second number of iterations (denoted as N2). The result is output volume image data (denoted as V2_OUT 170) associated with the second resolution level (RES2).

### Comparison

Examples of the present disclosure may be implemented to improve the computational efficiency of image reconstruction, leading to faster clinical workflows and more timely clinical decision-making. Examples of the present disclosure should be contrasted against conventional iterative reconstruction approaches that involve processing an entire high-resolution dataset. A comparison will be explained using FIGs. 2A-B. Here, FIG. 2A is a schematic diagram illustrating conventional iterative image reconstruction, whereas FIG. 2B is a schematic diagram illustrating a first example of iterative image reconstruction with upscaling.

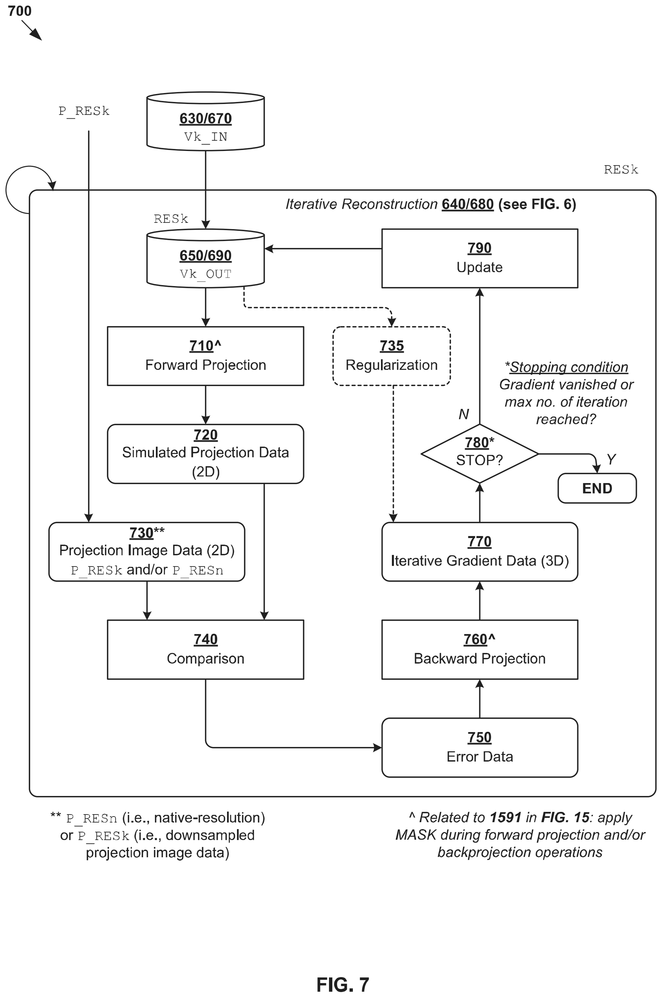

In FIG. 2A, iterative image reconstruction (see 220) may be performed to generate high-resolution volume image data (see 230) based on an initial volume (see 210). Since reconstruction time depends on voxel grid resolution, the high-resolution iterative reconstruction in FIG. 2A may be inherently slow and computationally intensive. This is because iterative image reconstruction generally relies on the execution of a pair of projectors (i.e., forward-projector and back-projector) in an iterative process. The pair of projectors work together to update volume image data progressively until convergence is achieved or a stopping condition/criterion, such as convergence or maximum number of iterations reached, is met. See example projectors at 710 and 760 in FIG. 7 (to be described below).

In practice, the forward-projector simulates the passage of X-rays through the volume to produce simulated projection data, while the backprojector uses the projection data to update information within the volume domain. The implementation of forward projection and backprojection operations usually constitutes most of the computational time required for iterative image reconstruction. The runtime of these projectors is directly proportional to the overall number of volume voxels and the number of corresponding detector pixels. The number of floating point operations and memory access operations for each of these projectors may be in the order of the number of voxels in the image domain multiplied by the number of pixels in the projection domain. Accordingly, the computational demand increases with higher resolution. For a large image volume with fine resolution, the computational complexity may escalate rapidly.

In contrast, the first aspect of the present disclosure provides an iterative image reconstruction approach that is multiresolution in the time domain, meaning that the resolution of the volume image data evolves over time and varies at different stages of the reconstruction. Examples of the present disclosure may be implemented to reduce computational time by leveraging an observation that earlier iterations in an iterative reconstruction process recover low-frequency features first, whereas later iterations contribute to higher-frequency features. Additionally, examples of the present disclosure may be implemented to reduce clinical workflow time, such as by generating user interface (UI) views to provide lower-resolution V1_OUT 130 to a user (e.g., clinician) as a preview, followed by higher-resolution V2_OUT 170.

In the example shown in FIG. 2B, lower-resolution data is processed during a first time period (see 280), followed by higher-resolution data during a second time period (see 290). During a first resolution refinement stage (see 250), iterative reconstruction may be performed to generate V1_OUT 130 associated with RES1 based on initial volume image data (V1_IN) 240 and first downsampled projection image data (P_RES1) 110. During an upscaling stage (see 260), V1_OUT 130 may be upscaled into V2_IN 150 associated with RES2. During a final resolution refinement stage (see 270), iterative reconstruction may be performed to refine V2_IN 150 into output volume image data (V2_OUT) 170. Final stage 270 may be performed based on second downsampled projection image data (denoted as P_RES2) that is generated by downsampling native-resolution P_RESn 105 in FIG. 1. The downsampling of P_RESn 105 will be explained further using FIG. 6 below.

Any suitable RES1 and RES2 may be used in practice. For example in FIG. 2B, RES1 may be 256x256x193, while RES2 512x512x128. In this case, V2_IN 150 has 2x2x2 = 8 times more voxels compared to V1_OUT 130. This multi-stage approach to image reconstruction may lead to quicker convergence, reduced processing time, and lower computational resource requirements while still achieving high-resolution results. This may ultimately improve patient outcomes and increase the number of patients treatable on a single radiation therapy system.

### Multiple (K ≥ 1) upscaling stages

The case of *K* = 2 upscaling stages is shown in FIG. 3, which is a schematic diagram illustrating a second example (see 300) of iterative image reconstruction with multiple upscaling stages. The example in FIG. 3 includes *K* + 1 = 3 resolution levels: lower-resolution RES1 ("first resolution level") for *k* = 1, intermediate-resolution RES2 ("second resolution level") for *k* = 2, and higher-resolution RES3 ("third resolution level") for *k* = 3. This way, multi-stage iterative reconstruction may be performed to first recover low-frequency features associated with RES1, followed by intermediate-frequency features associated with RES2, and finally finer details associated with RES3. This should be contrasted against conventional approaches that only involve iterative reconstruction using one resolution level, as explained using FIG. 2A.

During first stage 301 in FIG. 3, resolution refinement (see 315) may be performed for N1 iterations to generate V1_OUT 320 associated with lower-resolution RES1 based on initial volume image data (V1_IN) 310 and first downsampled projection image data (P_RES1) 110 associated with RES1. During second stage 302, upscaling (see 325) may be performed to transform V1_OUT 320 into V2_IN 330, which is associated with intermediate-resolution RES2. During third stage 303, resolution refinement (see 335) may be performed for N2 iterations to generate V2_OUT 340 based on V2_IN 330 and second downsampled projection image data (P_RES2) associated with RES2. Operations 315, 325 and 335 in FIG. 3 are related to corresponding operations 250-270 in FIG. 2B.

Additionally, during fourth stage 304, upscaling (see 345) may be performed to transform V2_OUT 340 into V3_IN 350 associated with higher-resolution RES3. Further, during fifth stage 305, resolution refinement (see 355) may be performed for N3 iterations based on V3_IN 350 and third downsampled projection image data (P_RES3) associated with RES3. The downsampling of native-resolution P_RESn to generate downsampled projection image data (P_RES1, P_RES2, P_RES3) will be explained using FIG. 6 below. The final/output volume image data may be denoted as V(*K +* 1)_OUT, in this case V3_OUT 360 for *K +* 1 = 3. As such, for *K =* 2, a total of 2*K +* 1 = 5 stages may be performed to generate the output, including *K* = 2 upscaling stages and *K* + 1 = 3 resolution refinement stages in FIG. 3.

In one example, RES3 = 512x512x256 voxel grid, which has eight times more voxels than RES2 = 256x256x128 voxel grid. Further, RES2 has eight times more voxels than RES1 = 128x128x64 grid. Using hivx = total number of high-resolution voxels, N1 = 10 iterations may be performed during first stage 301 to process total relative voxels = 10/8/8 hivx. Next, N2 = 5 iterations may be performed during third stage 303 to process 5/8 hivx. Finally, N3 = 5 iterations may be performed during fifth stage 305 to process 5 hivx. Compared to performing 10 iterations in RES3 only, the multi-stage image reconstruction process in FIG. 3 may lead to a theoretical time saving of 1-(10/8/8+5/8+5)/10 = 42.2% using two upscaling stages (*K* = 2). Even when one upscaling stage is implemented (*K* = 1), a theoretical time saving of 1-(10/8+5)/10 = 37.5% may be achieved.

Using examples of the present disclosure, iterative reconstruction may be accelerated to reduce the time required to obtain high-quality images, which is crucial in time-sensitive applications like medical diagnostics. Performing iterative reconstruction in the lower resolution level(s) also reduces computational demand, making the process more efficient and feasible on standard hardware. An accelerated process for iterative reconstruction also improves clinical workflow efficiency, allowing for more timely decision-making.

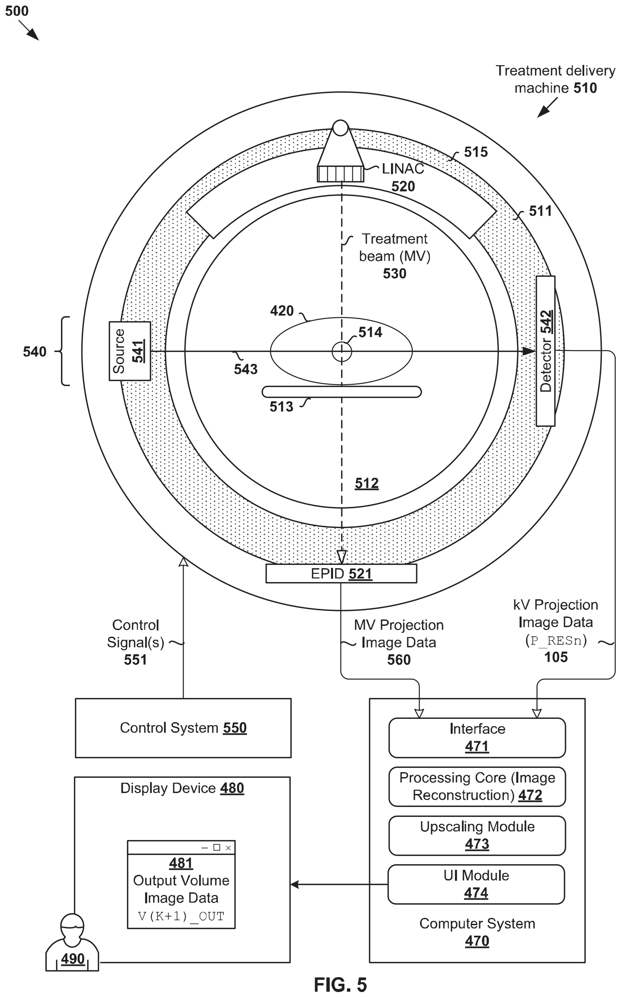

Throughout the present disclosure, it should be understood that V*k*_OUT and V(*k* + 1)_IN may represent different resolution levels, but the same volume of physical space in FIGs. 1-15. For example, in FIG. 2B and FIG. 3, the outer dimension of V1_OUT 130/320 may be the same as that of V2_IN 150/330. In FIG. 3, the outer dimension of V2_OUT 340 may be the same as that of V3_IN 350, etc. Examples of the present disclosure may be implemented using any suitable space-filling representation in the spatial domain, such as cubic voxels, rectangular cuboids, triangular prisms, hexagonal prisms, truncated octahedron, rhombic dodecahedron, etc. Upscaling according to examples of the present disclosure may be performed in any arbitrary size and/or direction(s). For example, upscaling may be performed along any combination of one or more of a longitudinal axis, a transverse axis and a vertical axis. For example, rectangular cuboid voxels may be upsampled to any other grid with any suitable step in each direction. The upscaling process may vary for each level of upscaling, allowing for different resolutions and levels of detail to be achieved to meet the requirements of different applications. Detailed examples will be described below using FIGs. 5-11.

### Example radiation therapy systems

According to examples of the present disclosure, any suitable computer system may be configured to implement the first and second aspects of the present disclosure. Two examples will be discussed below. In a first example, a computer system (see 470 in FIG. 4) may be configured to perform image reconstruction with upscaling according to the first and/or second aspects during a pre-treatment phase of radiation therapy, such as for diagnosis and treatment planning purposes. High-quality reconstructed images are important for segmentation, which identifies and delineates a target tumor and surrounding healthy tissues. Based on the segmentation, an effective treatment plan may be developed to deliver radiation doses to the tumor while sparing the healthy tissues.

In a second example, a computer system (see 470 in FIG. 5) may be configured to perform image reconstruction with upscaling according to the first and/or second aspects during a treatment phase of radiation therapy. In practice, real-time or near-real-time volume image data (i.e., reconstructed images) enable clinicians to monitor and adjust the treatment delivery based on any detected changes in the patient's anatomy, tumor position and size. The output volume image data may also be used for patient positioning and target structure tracking during treatment, improving the precision and effectiveness of radiation delivery.

### (a) Pre-treatment phase

FIG. 4 is a schematic diagram illustrating example radiation therapy system 400 that includes computer system 470 to perform image reconstruction during a pre-treatment phase of radiation therapy. Depending on the desired implementation, system 400 may include additional and/or alternative components than that shown in FIG. 4. In this example, radiation therapy system 400 may include imaging system 410 to acquire native-resolution projection image data 105, control system 460 to control operations of imaging system 410 and computer system 470 to perform image reconstruction according to examples of the present disclosure. Display device 480 may be communicatively coupled with computer system 470 to display user interface (UI) views associated with volume image data generated by computer system 470. Imaging system 410 may include gantry 411 having opening 412 and patient support 413 for supporting patient 420 requiring radiation therapy.

Imaging system 410 may implement any suitable imaging modality for image data acquisition, such computed tomography (CT), positron emission tomography (PET), single photon emission computed tomography (SPECT), magnetic resonance imaging (MRI), magnetic resonance tomography (MRT), any combination thereof, etc. For example, when CT is used, projection image data 105 (e.g., planning CT scan) may include a series of 4D projection images or slices (e.g., CT slices), each representing a cross-sectional view of the patient's anatomy. For treatment planning, projection image data 105 may include 4D volumetric CT data that is used (sometimes in combination with 5D CT) to estimate the motion range of target structure(s). For example, spectral CT data (e.g., dual energy CT (DECT) and photon counting CT) may be acquired instead or additionally to provide access to various quantities at the planning stage.

In the example in FIG. 4, gantry 411 has a ring-based configuration. In an alternative example, gantry may have a C-arm configuration. Imaging system 410 may include imaging or radiation source 430 (e.g., X-ray source) to project imaging beams 450 towards detector 431 having pixel detectors disposed opposite of source 430. Control system 460 may be electrically coupled to gantry 411 to control the latter's operations using control signal(s) 461. Radiation source 430 may be configured to generate any suitable beam, such as fan beam, etc. During an imaging procedure, gantry 411 may be rotated about opening 412 while radiation source 430 generates and directs X-ray beam(s) 450 along a projection line towards patient 420 and detector 431. Detector 431 may measure the X-ray absorption and produce a voltage proportional to the intensity of incident X-rays. The voltage may be read and digitized to generate projection image data 105. Projection image data 105 may include image data acquired at different gantry angles.

According to examples of the present disclosure, computer system 470 may obtain native-resolution projection image data (P_RESn) 105 from imaging system 410 and perform image reconstruction to generate output volume image data for display on display device 280. In the example in FIG. 4, computer system 470 may include interface 471 to interact with imaging system 410 to obtain projection image data 105; processing core 472 to perform downsampling and *K* + 1 resolution refinement stages; upscaling module/unit 473 to perform *K* upscaling stage(s), and UI module 474 to generate and display UI views 481 on display device 480. UI module 474 may also be configured to receive input data from user 490 (e.g., clinician). Computer system 470 may include any alternative and/or additional components not shown in FIG. 4.

### (b) Treatment phase

FIG. 5 is a schematic diagram illustrating example radiation therapy system 500 that includes computer system 470 to perform image reconstruction during a treatment phase of radiation therapy. Depending on the desired implementation, radiation therapy system 500 may include additional and/or alternative components than that shown in FIG. 5. In this example, radiation therapy system 500 may include treatment delivery machine 510 to deliver treatment to patient 420; control system 550 to control operations of machine 510; and computer system 470 to perform image reconstruction according to examples of the present disclosure.

Treatment delivery machine 510 may include gantry 511 that is rotatable about opening 512 and patient support 513 (e.g., treatment couch) for supporting patient 420. Note that gantry 511 may have a ring-based configuration (shown in FIG. 5) or C-arm configuration (not shown). Treatment delivery machine 510 may include a radiation source in the form of linear accelerator (LINAC) 520 as well as an imager/detector in the form of mega-electron volts (MV) electronic portal imaging device (EPID) 521. LINAC 520 may be configured to generate and direct treatment beam 530 towards isocenter 514 through a PTV associated with patient 420 as gantry 511 is rotated through a treatment arc during VMAT. In practice, treatment beam 530 may be within a high-energy range, such as 1 MV or greater. Radiation therapy may be delivered as a fractionated treatment, where the total radiation dose to be delivered to a tumor is divided into smaller "fractions." This is to allow healthy cells to recover in between fractions from the damage caused by radiation, while tumor cells that are less efficient at recovering may accumulate damage.

Treatment delivery machine 510 may further include on-board imaging system 540 to facilitate kilovolt (kV) imaging during application of MV treatment beam 530. Any suitable image modality or modalities may be used, such as SE or DE CBCT, etc. Imaging system 540 may include at least one kV imaging source 541 and at least one kV imager 542. Compared to LINAC 520, kV imaging source 541 may be capable of producing imaging or diagnostic energy in the range of kV. During treatment delivery, control system 560 may configure kV imaging source 541 to emit and direct kV imaging beam 543 towards imager 542, thereby generating projection image data 105 in the form of kV projection image data. Although described with reference to MV LINAC 520 and MV treatment beam 530, it should be understood that any additional or alternative treatment delivery technique(s) may be used. For example, a proton treatment machine that includes a kV imaging system may be used instead.

Computer system 470 in FIG. 5 may be communicatively coupled with imaging system 510 to obtain may obtain native-resolution projection image data (P_RESn) projection image data 105 from on-board imaging system 540 via interface 471 and perform image reconstruction according to examples of the present disclosure. Computer system 470 may further include processing core 472 to perform downsampling and *K* + 1 resolution refinement stages; upscaling module/unit 473 to perform *K* upscaling stage(s), and UI module 474 to generate and display UI views 481 on display device 480. UI module 474 may also be configured to receive input data from user 490 (e.g., clinician). Computer system 470 in FIGs. 4-5 may be implemented using a physical machine and/or virtual machine that is deployed in a cloud-based environment (i.e., not located in the same physical location as imaging system 410/540).

### Example resolution refinement stage

FIG. 6 is a flowchart of example detailed process 600 for computer system 470 to perform iterative image reconstruction with upscaling. Example process 600 may include one or more operations, functions, or actions illustrated by one or more blocks, such as 605 to 695. Depending on the desired implementation, various blocks may be combined into fewer blocks, divided into additional blocks, and/or eliminated. The example in FIG. 6 may be performed using computer system 470 in FIGs. 4-5.

The example in FIG. 6 may be implemented for any suitable *K* ≥ 1. For the *k^{th}* resolution refinement stage (see 640), input = V*k*_IN (see 630) associated with RESk = *k^{th}* resolution level may be refined over multiple iterations into output = V*k*_OUT (see 650) associated with RES*k*. For the *k^{th}* upscaling stage (see 660), V*k*_OUT associated with lower-resolution RES*k* may be transformed into upscaled V(*k* + 1)_IN associated with higher-resolution RES(*k* + 1).

At 610 in FIG. 6, based on native-resolution projection image data P_RESn (see 605 in FIG. 6 and 105 in FIG. 1) acquired using imaging system 410/540 in FIG. 4 or 5, computer system 470 may perform downsampling to generate downsampled projection image data (denoted as P_RES*k*; see 620) for *k* = 1,...,*K*+1. For resolution level = RESk, P_RES*k* 620 may be generated by downsampling P_RESn 605 using any suitable downsampling factor, such as *f*1 = *a*^{*n*-*k*} using any suitable base *a* (e.g., 2, 3 or 4, etc.). Here, P_RES*k* represents a down-sampled (binned) version of native-resolution P_RESn 605. Any suitable downsampling approach may be implemented, such as value binning that is a relatively straightforward and fast process, etc. Based on P_RESn 605, a sequence of downsampling or binning operations may be performed to generate P_RES*k* 620, such as from highest-resolution P_RES(*K*+1) to lower-resolution P_RES1. Note that RES1 ≤ RES *k* ≤ RES (*k +* 1) ≤ RES (*K +* 1) ≤ RESn, i.e., the native resolution level is the highest possible resolution level. In the case of P_RES(*K*+1) = P_RESn, P_RESn may be used as input data for an iterative reconstruction stage.

At 640 in FIG. 6, computer system 470 may implement resolution refinement stage *k* to generate output data = V*k*_OUT (see 650) associated with RESk. At 641, iterative reconstruction may be performed for N*k* = number of iterations based on the following input data: (a) downsampled projection image data (P_RES*k*) 620 and (b) initial volume image data denoted as V*k*_IN 630. For *k* = 1, V1_IN 630 may be a zero or prior low-resolution volume that is generated using any suitable approach. At 642, it is determined whether a stopping condition has been met, such as whether gradient data (see 770-780 in FIG. 7) has vanished, whether a user-configurable maximum number of iterations have passed, etc.

Any suitable algorithm for iterative reconstruction may be implemented at block 640 to improve the quality of reconstructed images through repeated refinement over multiple iterations. Any suitable optimization techniques for fast convergence may be used during the iterative reconstruction, such as subsets or momentum. Also, different regularization approaches may be employed to facilitate stabilization of the convergence as well as noise suppression in the output volume. An example implementation of block 640 will be explained using FIG. 7 below. Additional implementation details may be found in United States Patent No. 11,173,324 entitled "Iterative reconstruction in image-guided radiation therapy," which is incorporated herein by reference in its entirety.

FIG. 7 is a flowchart of an example process (see 700) for computer system 470 to implement a resolution refinement stage in the example in FIG. 6. First, an estimate of the output data = V*k*_OUT 650 may be initialized or obtained, such as based on an initial volume estimate (e.g., V1_IN 310 in FIG. 3) or the output data from a previous upscaling stage. Then, at 710 in FIG. 7, V*k*_OUT 650 may be subjected to forward projection, where 2D simulated projection data 720 is generated by projecting V*k*_OUT 650 onto the same or similar detector geometry used during the actual acquisition of projection image data 110.

At 740 in FIG. 7, simulated projection data 720 may be compared with measured projection data 730 to determine difference data (see 750) representing any error between them. Measured projection data 730 may be native-resolution projection data (P_RESn) 605 or downsampled projection data (P_RESk) 620 in FIG. 6. Next, at 760, difference data 750 may be subjected to backprojection to generate iterative gradient data (3D) 770. Backprojection 760 is an inverse or approximate inverse process to that of forward projection 710.

At 780, it is determined whether a stopping condition has been met, such as whether gradient data 770 has vanished (i.e., convergence achieved) or a maximum number of iterations has been reached. If yes, the iterative process ends. Otherwise, at 790, Vk_OUT 650 may be updated based on gradient data 770 to reduce the error, and the cycle of forward projection and backprojection continues iteratively. Depending on the desired implementation, regularization (see 735) may be performed on V*k*_OUT 650 and/or gradient data 770, such as stabilizing (counterweight) data-fidelity to facilitate the convergence of gradient data 770. Note that regularization may be performed in different sections of the example in FIG. 7.

### Example upscaling stage

Referring to FIG. 6 again, at 660, computer system 470 may implement upscaling stage *k* to transform (a) input data = V*k*_OUT 650 associated with lower-resolution RES*k* into (b) output data = V(*k* + 1)_IN associated with higher-resolution RES(*k* + 1) based on an upscaling factor (*f*2). Here, V(*k* + 1)_IN 670 serves as input data to a subsequent resolution refinement stage *k +* 1. Any suitable *f*2 may be used, such as 2, 3 or 4, etc. An example implementation of block 660 will be explained using FIG. 8, which is a flowchart of an example process for computer system 470 to implement an upscaling stage in the example in FIG. 6.

At 801-803 in FIG. 8, block 660 may include upscaling input data = Vk_OUT 650 along one or more of the following axes: (a) Z-axis = longitudinal axis representing the depth of the volume image data, (b) X-axis = transverse or horizontal axis representing the width of volume image data, and (c) Y-axis = sagittal or vertical axis representing the height of the volume image data. Upscaling along any combination of these spatial dimensions helps capture finer details and edges within the volume image data, leading to a clearer differentiation between a target structure and its surrounding healthy tissues. For example, upscaling along the Z-axis may be performed to increase the resolution in the longitudinal direction, thereby improving the longitudinal (out-of-plane) resolution level. Upscaling along the X-axis increases the resolution in the horizontal direction, providing more detail and clarity in the width of the volume. Upscaling along the Y-axis increases the resolution in the vertical direction, allowing for more precise visualization of structures and details that extend vertically.

Depending on the desired implementation, at least one of the following upscaling approaches may be performed: bilinear upscaling (see 810 in FIG. 8), cubic or bicubic upscaling (see 820), and upscaling using an AI engine (see 830). In practice, bilinear upscaling may include performing linear interpolation to calculate new pixel values based on the weighted average of the four nearest neighboring pixels. Cubic or bicubic upscaling may include performing cubic interpolation to calculate new pixel values based on more nearest neighboring pixels, such as 16 neighboring pixels (i.e., four in each direction), etc. In general, the upscaling may include performing interpolation to calculate new pixel values based on any combination of any neighboring pixels. Compared to bilinear upscaling, cubic upscaling provides fewer artifacts and smoother gradients/transitions, resulting in higher-quality images. Any additional and/or alternative upscaling technique(s) may be used.

In the example in FIG. 8, AI engine 830 may include a hierarchy of multiple (*X*) processing layers (denoted as *A*₁ to *A_{X}*), such as an input layer, an output layer, and multiple (i.e., two or more) "hidden" layers between the input and output layers. The processing layers (*A*₁ to *A_{X}*) are associated with respective weight data (*w*₁ to *w_{X}*)*.* During training, AI engine 830 may learn weight data (*w*₁ to *w_{X}*) to perform upscaling by transforming input = V*k*_OUT 650 associated with lower-resolution RES*k* into output = V(*k* + 1)_IN 650 associated with higher-resolution RES(*k* + 1).

As used herein, the term "AI engine" may refer to any suitable hardware and/or software components of a computer system that are capable of executing algorithms according to any suitable AI model(s). An "Al engine" may be a machine learning engine based on machine learning model(s), deep learning engine based on deep learning model(s), etc. In general, deep learning is a subset of machine learning in which multilayered neural networks may be used for feature extraction as well as pattern analysis and/or classification.

Any suitable AI model(s) may be used to implement AI engine 830, such as convolutional neural network, recurrent neural network, deep belief network, generative adversarial network (GAN), autoencoder(s), variational autoencoder(s), long short-term memory architecture for tracking purposes, generative AI model, transformer network, or any combination thereof, etc. In practice, a neural network is generally formed using a network of processing elements (called "neurons," "nodes," etc.) that are interconnected via connections (called "synapses," "weight data," etc.). A processing layer of a convolutional neural network may be a convolutional layer, pooling layer, un-pooling layer, rectified linear units (ReLU) layer, fully connected layer, loss layer, activation layer, dropout layer, transpose convolutional layer, concatenation layer, attention layer, any combination thereof, etc. For example, convolutional neural networks may be implemented using any suitable architecture(s), such as UNet, LeNet, AlexNet, ResNet, VNet, DenseNet, OctNet, etc.

AI engine 830 may be trained using any suitable approach, such as supervised learning, unsupervised learning, semi-supervised learning, reinforcement learning, etc. For example, using supervised learning, AI engine 830 may be trained on a dataset of labeled examples in order to learn the relationship between (a) input data and (b) output data. Any suitable training data may be used, such as synthetic data, real patient data, or a combination of both. AI engine 830 may be trained using training data that is specific to patient 420, or a large variation of possible patients. For example, a patient-specific training strategy may tackle the issue of inter-patient and inter-tumor variations (e.g., tumor size, shape, location, motion).

Alternatively, using unsupervised learning, AI engine 830 may be trained on a dataset of unlabeled examples to learn patterns and relationships in the data without any prior knowledge of the output labels. In semi-supervised learning, both labeled and unlabeled data may be used. Semi-supervised learning is useful in situations where there is a large amount of unlabeled data available, but it might be too expensive or difficult to label all the data. In reinforcement learning, AI engine 830 may learn to perform image reconstruction by trial and error where it is rewarded for taking actions that lead to desired outcomes and penalized for taking actions that lead to undesired outcomes.

After upscaling, at 680 in FIG. 6, computer system 470 may implement resolution refinement stage *k* + 1 to generate output data = V(*k* + 1)_OUT 690 associated with higher-resolution RES(*k* + 1). Block 680 may be performed based on (a) V(*k* + 1)_IN 670 associated with RES(*k* + 1), which is the output of upscaling stage 660 and (b) downsampled projection image data P_RES(*k* + 1) 675. At 681, iterative reconstruction may be performed for N(*k* + 1) = number of iterations based on input data = V(*k* + 1)_IN 670 and P_RES(*k* + 1) 675. In the case of *k = K* and RES(*K* + 1) = RESn, block 1590 may be performed based on P_RESn (i.e., no downsampling required when the highest resolution level is the native resolution level). At 682, it is determined whether a stopping condition has been met. Block 680 may be implemented using the example in FIG. 7, which has been explained above and will not be repeated here for brevity.

At 695 in FIG. 6, computer system 470 may determine whether *k = K* in that all *K* upscaling stage(s) *and K +* 1 resolution refinement stages have been completed. If not, computer system 470 may set *k = k +* 1 and perform block 660 again. Otherwise, in response to determination that *k = K,* example process 600 in FIG. 6 stops.

### Iterative image reconstruction approaches

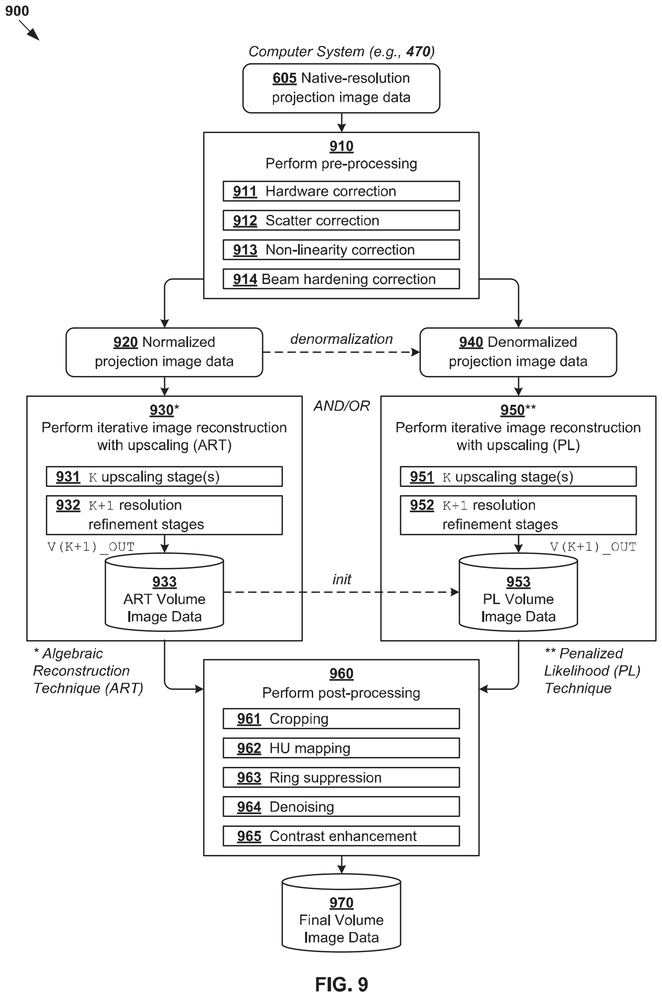

Depending on the desired implementation, the multi-stage iterative reconstruction process in FIG. 6 may be performed to support multiple image reconstruction techniques. Some examples will be discussed using FIG. 9, which is a flowchart of an example process for a computer system to perform iterative reconstruction using an algebraic reconstruction technique (ART) and/or a penalized likelihood (PL) technique. Example process 900 in FIG. 9 may include one or more operations, functions, or actions illustrated by one or more blocks, such as 910 to 960. Depending on the desired implementation, various blocks may be combined into fewer blocks, divided into additional blocks, and/or eliminated. The example in FIG. 9 may be performed using computer system 470 in FIGs. 4-5, such as using processing core(s) 472, upscaling module 473, etc.

### (a) Pre-processing

At 910 in FIG. 9, based on projection image data 110 (e.g., native-resolution projection data), computer system 470 may perform pre-processing prior to image reconstruction. Any suitable pre-processing operation(s) may be performed at block 910, such as defect correction, scatter correction, non-linearity correction, beam hardening correction, or any combination thereof. Pre-processing may be performed on a projection basis while data acquisition is still running during a scan.

At 911 in FIG. 9, hardware defect correction may involve identifying and correcting any defect(s) in the detector system (e.g., detector 431 in FIG. 4 or 542 in FIG. 5). For example, defects (e.g., dead pixels or areas with inconsistent responses) may cause artifacts in projection image data 110 as well as the resulting volume image data. At 912 in FIG. 9, scatter correction may be performed to mitigate the effects scattered radiation from projection image data 110, thereby enhancing its clarity and contrast. Scattered radiation, which occurs when X-rays deviate from their original path, may cause blurring and reduced contrast.

At 913 in FIG. 9, non-linearity correction may be performed to compensate for the non-linear response of the detector system. This is because detectors may not respond linearly to varying radiation intensities, causing distortions in projection image data 110. Correcting these non-linearities in projection image data 112 ensures a more accurate representation of the true distribution of the radiation. At 914 in FIG. 9, beam hardening correction may be performed to compensate for the effects of beam hardening, where lower energy X-rays are absorbed more than higher energy X-rays as they pass through a subject for polychromatic X-ray sources. By applying beam hardening correction, projection image data 110 may be adjusted to account for differential energy absorption, thereby improving image quality. In practice, attenuation may not necessarily drop as energy increases, such as when k-edges are taken into account, in which case the attenuation coefficient may suddenly jump up with slightly higher energy. Also, note that not all sources used for transmission imaging need to be polychromatic.

### (b) Iterative reconstruction

At 930 in FIG. 9, computer system 470 may perform iterative reconstruction according to a first approach in the form of ART. In practice, ART may refer generally to an iterative reconstruction technique that solves a system of linear equations derived from projection data, updating values iteratively. For example, a volume image data estimate may be updated voxel by voxel in the reconstruction space, using the contributions from each projection. Using the example in FIG. 6, *K* upscaling stage(s) and *K* + 1 resolution refinement stages (see 931-932) may be performed to generate output ART volume 933 based on normalized projection image data 920.

Alternatively or additionally, at 950 in FIG. 9, computer system 470 may perform iterative reconstruction according to a second approach in the form of PL. In practice, PL is an approach that maximizes a likelihood function with an added penalty term to achieve desired properties (e.g., smoothness) in the reconstructed image. In practice, PL may also be referred to as model-based iterative reconstruction (MBIR) or statistical iterative reconstruction (SIR). Using the example in FIG. 6, *K* upscaling stage(s) and *K* + 1 resolution refinement stages (see 951-952) may be performed to generate output PL volume 953 based on denormalized projection image data 940. ART volume image data 933 may be used to initialize the process at block 950.

Note that normalized projection image data 920 may be used at block 930 because ART generally operates in attenuation value space, i.e., the same space as ART volume image data 933. This may simplify the reconstruction process but may not fully leverage the statistical properties of the data. In contrast, denormalized projection image data 940 may be used at block 950 because PL generally operates in intensity space. In this case, denormalization may be performed to more accurately model the statistical nature of the acquisition system (e.g., FIGs. 4-5), such as using Poisson distribution models, etc.

### (c) Post-processing

At 960 in FIG. 9, computer system 470 may perform post-processing based on ART volume image data 933 and/or PL volume image data 953 to generate final volume image data 970. Any suitable post-processing operation(s) may be performed at block 960, such as cropping, Hounsfield Unit (HU) mapping, ring suppression, denoising, contrast enhancement, etc. Cropping (see 961) may be performed to remove any unnecessary or irrelevant parts of the image, focusing on a preferred area of interest, and reducing the amount of data stored and processed.

HU mapping (see 962) may be performed to convert the raw reconstructed data into standardized Hounsfield Units, which are used to quantify the radiodensity of tissues. In practice, CBCT imaging systems for radiation therapy may also produce electron-density (ED) values, not only HU. Ring suppression (see 963) may be performed to reduce or eliminate ring artifacts that may appear due to imperfections in the detector system or inconsistencies in the data acquisition process. Denoising (see 964) may be used to reduce image noise within the reconstructed volume. Contrast enhancement (see 965) allows visualization of differently absorbing structures in a single view without adaptation of window/level. One or more of these post-processing steps may be implemented to enhance usability and accuracy, contributing to better clinical outcomes.

### Example UI views

As used herein, the term "UI" or "UI view" may refer generally to a set of UI elements that may be generated and displayed on a display device. The term "UI element" may refer generally to graphical (i.e., visual) and/or textual element that may be displayed on a display device, such as shape (e.g., circle, rectangle, ellipse, polygon, line, etc.), window, modal, panel or pane, button, check box, menu, dropdown box, editable grid, section, side bar, slider, text box, text block, toggle switch (on/off button), or any combination thereof. UI views may be displayed side by side or nested inside of each other to create more complex layouts. The term "interacting" may refer generally to a range of actions for a user to engage with a UI view, such as viewing, clicking, swiping, changing the orientation and/or size associated with the content of the UI view, annotating, etc. The term "display device" (e.g., see 480 in FIGs. 4-5) may refer generally to any suitable hardware component for presenting visual information to a user, such as a monitor, touchscreen, etc.

### (a) First UI view

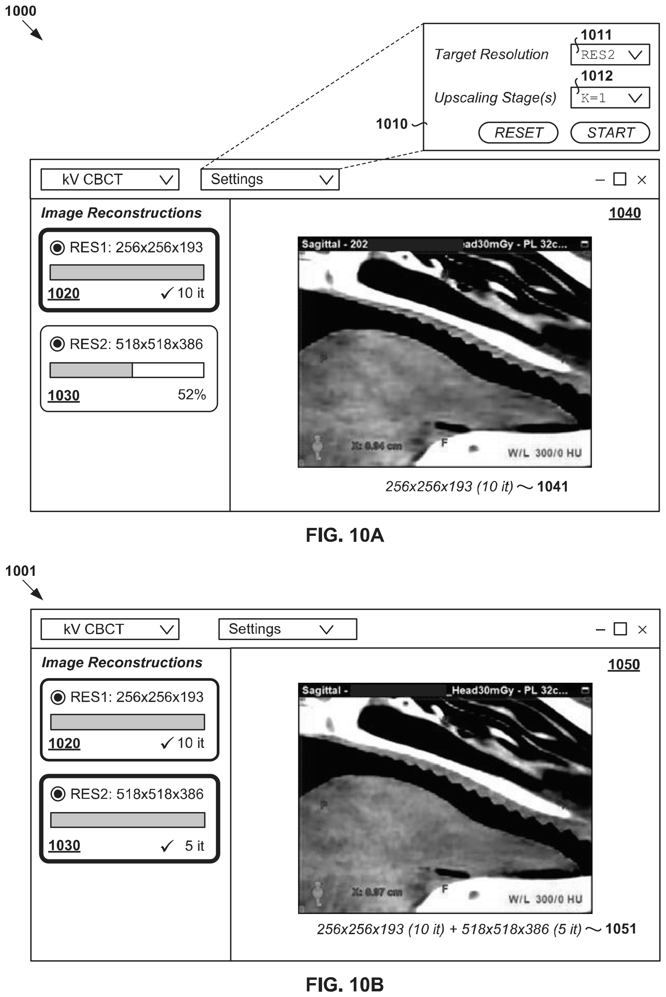

FIG. 10A is a schematic diagram illustrating first example UI view 1000 for user 490 to interact with first volume image data. In this example, computer system 470 (e.g., using UI module 474) may generate first UI view 1000 to include a UI element (see 1010) for user 490 to configure various settings relating to iterative image reconstruction with upscaling. At 1011, a target resolution may be configured, such as RES2 = 518x518x386. At 1012, the number of upscaling stage(s) may be configured, such as K = 1. FIG. 10A will be explained using the case of *K* = 2 in FIG. 2B.

At 1020-1030 in FIG. 10A, computer system 470 may generate first UI view 1000 to include multiple status indicators associated with *K* + 1 resolution refinement stages. First status indicator 1020 may be generated and dynamically updated to indicate the progress (e.g., 100%) of first resolution refinement stage 250 in FIG. 2B that generates V1_OUT 130. Second status indicator 1030 may be generated and dynamically updated to indicate the reconstruction progress (e.g., 62%) of second resolution refinement stage 270 that generates V2_OUT 170 in FIG. 2B. Any suitable status indicator may be generated and displayed, such as progress bars, charts, graphs, meters, tabs with built-in progress bar, and any other type of visual feedback.

Once lower-resolution V1_OUT 130 is generated, computer system 470 (e.g., using UI module 474) may generate and display first UI view 1000 on display device 480 for user 490 to interact with V1_OUT 130. At 1040 in FIG. 10A, first UI view 1000 may show any suitable orientation(s) or perspective(s) of V1_OUT 130, such as a sagittal view. Other example views include transversal view, frontal view, 3D view, etc. Additionally, at 1041, first UI view 1000 may specify at least one of the following: (a) RES1 = 256x256x193 and (b) the total number of iterations performed, i.e., N1 = 10 iterations associated with RES1. Lower-resolution V1_OUT 130 may be presented to user 490 as a preview before higher-resolution V2_OUT 170 is generated.

### (b) Second UI view

FIG. 10B is a schematic diagram illustrating second example UI view 1001 for user 490 to interact with output volume image data. Once higher-resolution V2_OUT 170 is generated, computer system 470 (e.g., using UI module 474) may generate and display second UI view 1001 on display device 480 for user 490 to interact with V2_OUT 170. At 1050 in FIG. 10B, second UI view 1001 may show any suitable orientation(s) or perspective(s) of V2_OUT 170, such as a sagittal view. Additionally, at 1051, second UI view 1001 may specify at least one of the following: (a) RES2 = 518x518x386 and (b) the total number of iterations performed, i.e., N1 = 10 iterations associated with RES1 = 256x256x193 plus N2 = 5 iterations associated with RES2.

### (c) Live switching

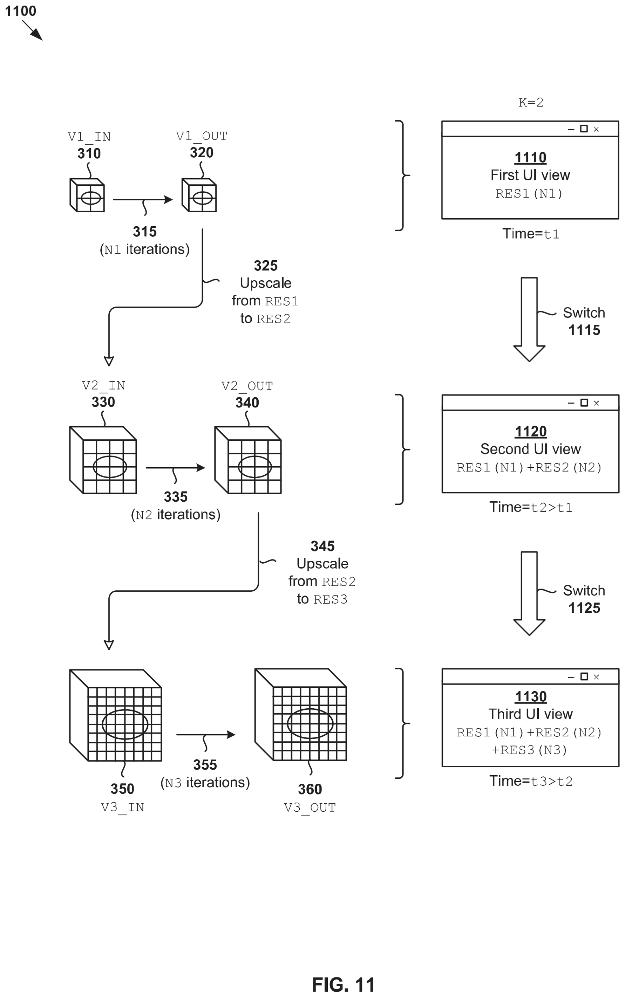

The examples in FIGs. 10A-B may be extended to the case of *K* ≥ 2 upscaling stages. An example is shown in FIG. 11, which is a schematic diagram illustrating multiple UI views for displaying multiple sets of volume image data. FIG. 11 will be explained with reference to the example in FIG. 3. Once lower-resolution V1_OUT 320 is generated, computer system 470 may generate and display first UI view 1110 on display device 480 for user 490 to interact with V1_OUT 320. First UI view 1110 generated at time = t1 may identify the number of iterations performed, i.e., N1 iterations associated with RES1.

Next, once intermediate-resolution V2_OUT 340 is generated, first UI view 1110 may be switched (see 1115) to second UI view 1120 at time = t2. Here, computer system 470 may generate and display second UI view 1120 for user 490 to interact with V2_OUT 340. Second UI view 1120 may identify the number of iterations performed, i.e., N1 iterations associated with RES1 + N2 iterations associated with RES2.

Finally, once higher-resolution V3_OUT 360 is generated, second UI view 1120 may be switched (see 1125) to third UI view 1130 at time = t3. Here, computer system 470 may generate and display third UI view 1130 for user 490 to interact with V3_OUT 360. Third UI view 1130 generated at time = t3 may identify the number of iterations performed, i.e., N1 iterations associated with RES1 + N2 iterations associated with RES2 + N3 iterations associated with RES3.

Using examples of the present disclosure, a lower-resolution volume (Vk_OUT) may be presented to user 490 while a higher-resolution volume (V(k + 1)_OUT) is generated. The time between the availability of lower-resolution Vk_OUT and higher-resolution V(k + 1)_OUT may be utilized by user 490 to perform other tasks, such as scrolling to a preferred position in the reconstructed volume, adjusting a window/level, performing rough adjustments (e.g., rough matching tasks), etc. High-quality volume data is usually not required for rough adjustments.

### Second aspect: multiresolution image reconstruction with upscaling

Optionally, any or all features of the first aspect may be combined with any or all features of the second aspect.

According to a second aspect, methods and computer systems for multiresolution image reconstruction with upscaling are described. For example, the image reconstruction may use multiple resolutions in the spatial domain. Using examples of the present disclosure, multiresolution image reconstruction may be implemented using at least one upscaling stage (i.e., *K* ≥ 1) to reduce computational time associated with image reconstruction while accounting for truncation artifacts. In practice, truncation artifacts may occur when a small diameter X-ray beam occupies less than the entire cross-section of a patient. These artifacts may arise due to the limited beam width, which leads to incomplete coverage and partial data capture. Reducing such artifacts is desirable because they often compromise the accuracy and quality of image reconstruction, particularly in ROI(s) representing more important areas for diagnosis and treatment planning.

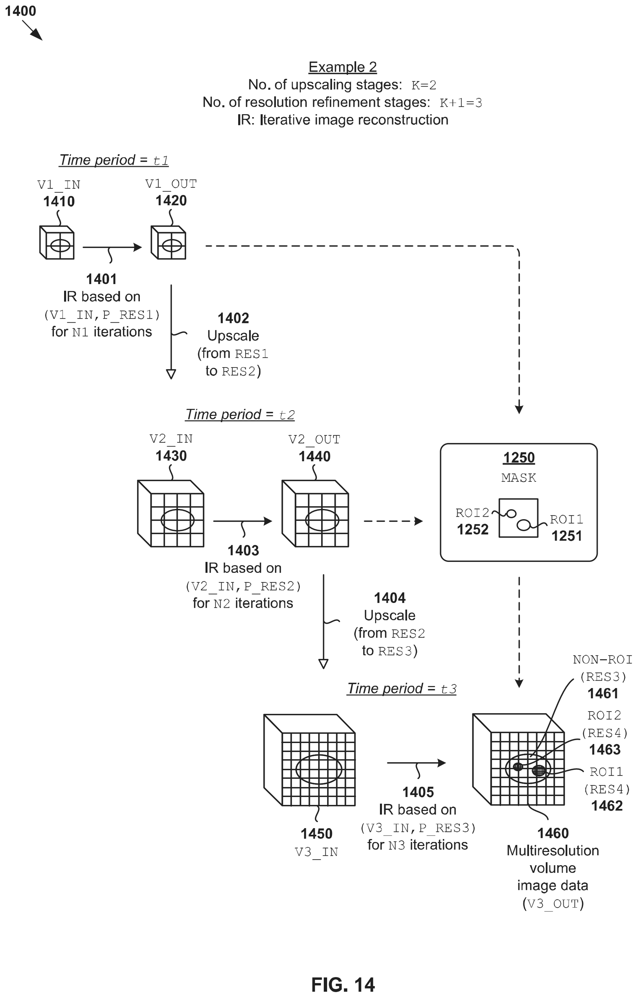

As will be described using FIGs. 12-15, examples of the present disclosure provide a multiresolution image reconstruction approach that is multiresolution in both time and spatial domains. In the time domain, the resolution level of the volume image data being estimated evolves over time and varies at different stages of the reconstruction process. This approach saves computational time by performing some (if not most) computations at a lower resolution level and higher-resolution computations to refine finer features. The final output (e.g., V2_OUT 1270 in FIGs. 12-13 or V2_OUT in FIG. 14) is multiresolution in the spatial domain, meaning that it includes voxels of different resolution levels. This allows one ROI in the output volume image data to be generated at a higher resolution level where more detail is needed, while another region that is less important may be generated at a lower resolution level. For example, a first ROI in the output volume image data may be generated at a higher resolution level while a second ROI in the output volume image data may be generated at a lower resolution level. Performing multiresolution image reconstruction with upscaling balances the need for high-quality imaging with the practical constraints of processing time and computational capacity.

As used herein, the term "multiresolution image reconstruction" may refer generally to an image reconstruction process for generating multiresolution volume image data. The term "multi resolution volume image data" may refer generally to volume image data associated with multiple resolution levels. The term "multiresolution iterative image reconstruction" may refer generally to an image reconstruction process that is performed iteratively for generating multiresolution volume image data. An example of the second aspect will be explained using FIG. 12, which is a flowchart of an example process (see 1200) for computer system 470 to perform multiresolution image reconstruction with upscaling for radiation therapy. Example process 1200 may include one or more operations, functions, or actions illustrated by one or more blocks, such as 1210 to 1270. Depending on the desired implementation, various blocks may be combined into fewer blocks, divided into additional blocks, and/or eliminated. Any suitable computer system may be configured to perform various examples of the present disclosure, such as computer system 470 in FIGs. 4-5.

At 1210 in FIG. 12, computer system 470 may obtain first volume image data (V1_OUT) associated with a first resolution level (RES1). Depending on the desired implementation, block 1210 may include computer system 470 generating V1_OUT 1210 based on projection image data (see 110 in FIG. 1) associated with a target structure of patient 420 requiring radiation therapy.

At 1220 in FIG. 12, computer system 470 may upscale V1_OUT 1210 associated with RES1 to generate second volume image data (V2_IN) 1230 associated with a second, higher resolution (RES2), i.e., RES2 > RES1. Block 1220 may be implemented using various examples in FIG. 8, such as bilinear upscaling, cubic or bicubic upscaling, AI engine for upscaling, etc.

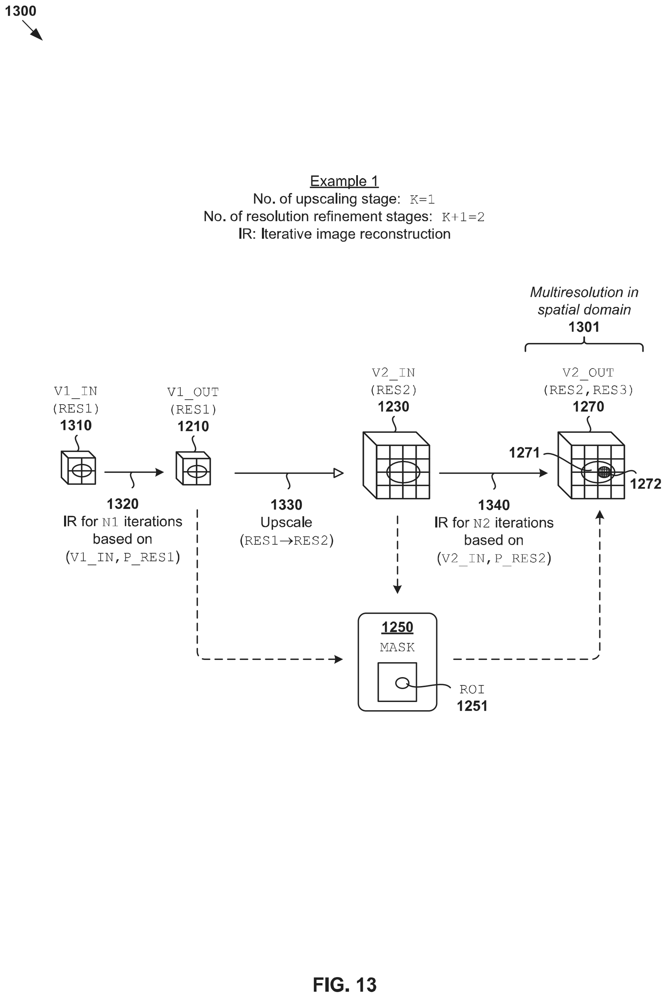

At 1240 in FIG. 12, computer system 470 may obtain mask image data 1250 (denoted as MASK) identifying at least one ROI 1251 associated with the target structure of patient 420. As used herein, the term "region of interest" or "ROI" (for example an ROI identified by the mask image data) may refer generally to a specific area in which higher-resolution image reconstruction is desired. Depending on the desired implementation, each ROI may be defined in the projection space and/or volume space. The term "mask image data" may refer to any suitable data for distinguishing an ROI from one or more other regions where higher-resolution image reconstruction is not desired. As will be described using FIG. 13, block 1240 may include computer system 470 generating MASK 1250, such as based on V1_OUT 1210, V2_IN 1230, projection image data 110, additional data (e.g., segmentation data), or any combination thereof.

For example, MASK 1250 may identify one or more ROIs representing areas, e.g. important areas, for medical diagnosis and treatment planning. Each ROI may be of any suitable size and shape, such as a 2D or 3D shape. ROI 1251 may be a region having a high degree of high-spatial frequency (HF) content, such as at boundaries between organs and bone. In practice, ROI 1251 may include tumor margins (e.g. edge or boundaries of a tumor) to accurately delineate the tumor from its surrounding healthy tissues, a region where small extensions of the tumor might be spreading into adjacent tissues, a vascular structure for understanding a tumor's blood supply, an enlarged or affected lymph node near the tumor, etc.

At 1260 in FIG. 12, computer system 470 may generate multiresolution volume image data (denoted as V2_OUT 1270) based on V2_IN 1230 and MASK 1250. Here, the term "multiresolution volume image data" may refer generally to volume image data having multiple resolution levels. In the example in FIG. 12, V2_OUT 1270 may include first voxels (see 1271) associated with RES2 outside of the ROI. V2_OUT 1270 may further include second voxels (see 1272) associated with a third, higher resolution level (RES3) within the ROI, where RES3 > RES2 > RES1.

### Example upscaling stages

Depending on the desired implementation, computer system 470 may be configured to perform one or more upscaling stages (i.e., *K* ≥ 1). The case of *K =* 1 (i.e., one upscaling stage) will be explained using FIG. 13, which is a schematic diagram illustrating a first example (see 1300) of multiresolution image reconstruction with one upscaling stage. The case of *K* = 2 (i.e., two upscaling stages) will be explained using FIG. 14, which is a second example (see 1400) of multiresolution image reconstruction with multiple upscaling stages. Implementation details that have been described using block 660 in FIG. 6 and blocks 810-830 in FIG. 8 are also applicable here.

### (a) One upscaling stage (K = 1)

In the example in FIG. 13, iterative reconstruction (see 1320) may be performed to generate V1_OUT 1210 associated with RES1 based on initial volume image data (V1_IN) 1310. During an upscaling stage (see 1330), V1_OUT 1210 may be upscaled into V2_IN 1230 associated with RES2. During a resolution refinement stage (see 1340), multiresolution iterative reconstruction may be performed to generate multiresolution volume image data (V2_OUT) 1270 based on V2_IN 1230 and MASK 1250. MASK 1250 may be generated to identify ROI 1251 based on V1_OUT 1210 and/or V2_IN 1230. This approach allows for the generation of V2_OUT 1270, which includes first voxels 1271 associated with RES2 outside of ROI 1251 and second voxels 1272 associated with higher-resolution RES3 within ROI 1251. Depending on the desired implementation, the iterative reconstruction process at block 1320 may be performed based on first downsampled projection image data (P_RES1) associated with RES1. Similarly, the iterative reconstruction process at block 1340 may be performed based on second downsampled projection image data (P_RES2) associated with RES2. As described using block 610 in FIG. 6, downsampled projection image data 620 that includes P_RES1 and P_RES2 may be generated by downsampling native-resolution P_RESn (see 605 in FIG. 6) from imaging system 410/540 using any suitable downsampling factor (*f*1).

### (b) Multiple upscaling stages (K = 2)

In the example in FIG. 14, iterative reconstruction (see 1401) may be performed for N1 iterations to generate V1_OUT 1420 associated with RES1 based on initial volume image data (V1_IN) 1410 and P_RES1. During a first upscaling stage (see 1402), V1_OUT 1420 associated with lower-resolution RES1 may be upscaled into V2_IN 1430 associated with intermediate-resolution RES2, where RES2 > RES1. During a resolution refinement stage (see 1403), iterative reconstruction may be performed based on V2_IN 1430 and P_RES2 for N2 iterations to refine V2_IN 1430 into V2_OUT 1440. During a second upscaling stage (see 1404), V2_OUT 1440 associated with intermediate-resolution RES2 may be upscaled to generate V3_IN 1450 associated with higher-resolution RES3.

During a final resolution refinement stage (see 1405), iterative reconstruction may be performed for N3 iterations to generate multiresolution volume image data (V3_OUT) 1460 based on V3_IN 1450, P_RES3 associated with RES3, and MASK 1250. Depending on the desired implementation, MASK 1250 may identify multiple ROIs, such as first ROI 1251 and second ROI 1252. MASK 1250 may be generated based on volume image data 1420/1440/1450, projection image data 110, additional data (e.g., segmentation data), or any combination thereof.

To provide more detail and finer granularity within ROIs 1251-1252, V3_OUT 1460 includes voxels that have been reconstructed at various resolution levels within the same spatial domain. First voxels 1461 outside of ROIs 1251-1252 are associated with RES3, while second voxels 1462-1463 within ROIs 1251-1252 are associated with RES4 > RES3. Note that first voxels 1462 may have a different resolution level compared to second voxels 1463. It should be understood that V*k*_OUT and V(*k* + 1)_IN may represent different resolution levels, but the same volume of physical space in FIGs. 12-15. For example, in FIGs. 13-14, the outer dimension of V1_OUT 1210/1420 may be the same as that of V2_IN 1230/1430. In FIG. 14, the outer dimension of V2_OUT 1440 may be the same as that of V3_IN 1450, etc.

### Example detailed process

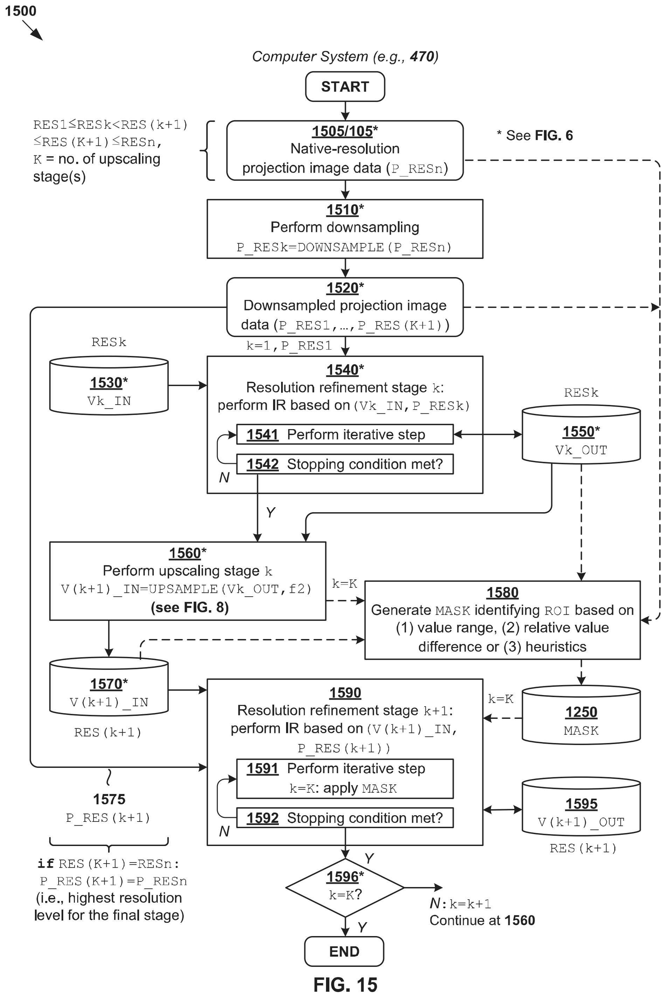

An example implementation will be described using FIG. 15, which is a flowchart of example detailed process 1500 for a computer system to perform multiresolution image reconstruction with upscaling. Example process 1500 may include one or more operations, functions, or actions illustrated by one or more blocks, such as 1505-1596. Depending on the desired implementation, various blocks may be combined into fewer blocks, divided into additional blocks, and/or eliminated. The example in FIG. 15 may be performed using computer system 470 in FIGs. 4-5.

### (a) First volume image data

Blocks 1505-1570 in FIG. 15 correspond with blocks 605-670 in FIG. 6, the description of which will not be repeated below for brevity. For block 1540 in FIG. 15, V1_OUT 1550 may be generated by performing iterative reconstruction for N1 iterations. Alternatively, V1_OUT 1210 may be generated by performing one of the following: filtered backprojection (FBP) algorithm, Feldkamp-David-Kress (FDK) algorithm and Defrise-Clark algorithm, iterative reconstruction with metal artifact reduction, four-dimensional (4D) image reconstruction, image reconstruction using an AI engine, etc.

In practice, FBP may involve applying a filter to projection image data 620 before backprojecting it onto an image plane. The FDK and Defrise-Clack algorithms extend the FBP algorithm to account for the geometry of cone-shaped X-ray beams. Iterative reconstruction with metal artifact reduction may involve reducing artifacts caused by a metal implant in a patient. 4D reconstruction may be implemented to extend the concept of 3D image reconstruction by incorporating a fourth dimension (e.g., time) to allow for the reconstruction of dynamic processes, such as respiratory or cardiac motion over time. This is particularly valuable in radiation therapy, where understanding the motion of tumors relative to surrounding tissues helps to improve treatment planning and delivery. Alternatively, an AI engine may be trained to generate V1_OUT 1550 based on any suitable projection image data.

Description of the FDK algorithm may be found in the following publication: "Practical cone-beam algorithm" by Feldkamp, L.A., Davis, L.C., Kress, J.W. in J. Opt. Soc. Am. 1(6) (1984). Description of the Defrise-Clack algorithm may be found in the following publications: "Cone-beam reconstruction by the use of Radon transform intermediate functions" by R. Clack, M. Defrise in J. Opt. Soc. Am 11 (2) Feb 1994 and "Direct Reconstruction of Cone-Beam Data Acquired with a Vertex Path Containing a Circle" by Noo. M. Defrise, R. Clack in IEEE Transactions on Image Processing 7 (6) Jun 1998. These publications are incorporated herein by reference.

Description relating to metal artifact reduction may be found in United States Patent Application Nos. US20230095240 and US20230100798, which are incorporated herein by reference. Description relating to 4D reconstruction may be found in the following publications: "A modified McKinnon-Bates (MKB) algorithm for improved 4D cone-beam computed tomography (CBCT) of the lung" by Star-Lack, J., et al. in Medical physics, 45(8), 3783-3799, 2018; and "A motion estimation and compensation algorithm for 4D CBCT of the abdomen" by Yoon, S., et al. in the 15th International Meeting on Fully Three-Dimensional Image Reconstruction in Radiology and Nuclear Medicine (Vol. 11072, pp. 59-63, May 2019), SPIE. Additional implementation details relating to iterative reconstruction may be found in the following publication: "Acuros CTS: A fast, linear Boltzmann transport equation solver for computed tomography scatter - Part II: System modeling, scatter correction, and optimization" by Wang, A., et al., in Medical Physics, 45(5), 1914-1925, 2018. These publications are incorporated herein by reference.

### (b) Upscaling

At 1560 in FIG. 15, computer system 470 may upscale (a) input data = V*k*_OUT 1550 associated with lower-resolution RES*k* to generate (b) output data = V(*k* + 1)_IN 1570 associated with higher-resolution RES(*k* + 1) based on any suitable upscaling factor (*f*2). Block 1560 may be implemented using the examples explained using FIG. 8, the details of which are not repeated here for brevity. For example, block 1560 may involve computer system 470 upscaling the input data along the Z-axis = longitudinal axis, X-axis = horizontal axis, Y-axis = vertical axis, or any combination thereof. Upscaling along the Z-axis increases the resolution in the longitudinal direction, thereby improving the longitudinal (out-of-plane) resolution level. Bilinear upscaling (see 810 in FIG. 8), cubic/bicubic upscaling (see 820), AI-based upscaling AI engine (see 830), or any combination thereof, may be implemented.

### (c) Mask image data generation

At 1580 in FIG. 15, computer system 470 may generate mask image data (MASK 1250/1585) identifying ROI(s) associated with a target structure of a patient. In one example, MASK 1250 may be in the form of a binary image that demarcates each ROI 1251. In this case, pixels or voxels within one ROI 1251 are assigned a flag value of 1 (i.e., TRUE), and all other pixels or voxels are assigned a value of 0 (i.e., FALSE). In another example, MASK 1250 may be a labeled image that uses labels to distinguish multiple ROIs within the image, such as "ROI1" for a first ROI, "ROI2" for a second ROI, etc. MASK 1250 may be used during image reconstruction to achieve higher resolution within ROI 1251. MASK 1250 may also include data identifying shape and/or contour associated with ROI 1251.

Depending on the desired implementation, MASK 1250 may be generated based on volume image data 1550/1570 in the 3D volume domain and/or projection image data 1505/1520 in the 2D projection domain. Additionally or alternatively, computer system 470 may obtain and process any suitable external data to generate MASK 1250. External data may include any suitable segmentation data relating to contoured surface(s) and/or edge(s) associated with a target structure. For example, the segmentation data may be used to trace the perimeter of a tumor to approximate its boundary. External data may also include user input data that may guide the identification of ROI 1251 for generating MASK 1250. For example, a UI view may be generated and displayed on display device 480 for user 490 to select ROI(s) within any suitable image data.

Any suitable approach may be used to generate MASK 1250, such as based on a value range associated with ROI 1251, relative value difference associated with ROI 1251, a heuristics-based approach for identifying ROI 1251, or any combination thereof. In a first example, computer system 470 identifying ROI 1251 based on a value range, such as by analyzing the pixel or voxel intensity values within Vk_OUT 1550. If ROI 1251 is associated with one or more values such as distinct intensity values, a threshold may be set to include values within a specific value range, thereby creating a mask (such as MASK 1250) where an ROI (such as ROI 1251) is marked or labelled.

In a second example, computer system 470 may identify ROI 1251 based on a relative value difference, which identifies edges or boundaries where there is a change in intensity values. This helps in isolating ROI 1251 that stands out from the background based on its contrast with neighboring regions. In a third example, computer system 470 may implement a heuristics-based approach by applying predefined rules and/or algorithms to determine ROI 1251, such as by detecting specific patterns, shapes, or sizes within volume image data 1550/1570 and/or projection image data 1505/1520. Depending on the desired implementation, MASK 1250 is updatable or modifiable during the subsequent multiresolution iterative image reconstruction process. For example, if *K* upscaling stages are configured and the value of the voxels within a parent voxel remains the same in the next iteration (i.e., low standard deviation between a child voxel and its parent voxel), the parent voxel may be masked as static for future refinement steps, thereby updating MASK 1250.

### (d) Multiresolution volume image data

At 1590 in FIG. 15, in response to determination that *k < K,* computer system 470 may perform iterative reconstruction to generate output data = V(*k* + 1)_OUT 1595. Iterative reconstruction may be performed according to the examples in FIG. 7. At 1591, iterative reconstruction may be performed for N*k* = number of iterations based on input data = V(*k* + 1)_IN 1570. At 1592, it is determined whether a stopping condition has been achieved, such as whether gradient data has vanished, whether a user-configurable maximum number of iterations have passed, etc. See also 1596.

In response to determination that *k = K* indicating the completion of all *K* upscaling stage(s), computer system 470 may perform multiresolution iterative reconstruction to generate output data = multiresolution volume image data V(*k* + 1)_OUT 1595 based on MASK 1250 and V(*k* + 1)_IN 1570. Depending on the desired implementation, block 1590 may be performed based on downsampled projection image data P_RES(*k* + 1) 1575. In the case of RES(*K* + 1) = RESn, block 1590 may be performed based on P_RESn (i.e., no downsampling required).

For *K* = 1 in the example in FIG. 13, the output multiresolution volume image data is V2_OUT 1270. For *K* = 2 in the example in FIG. 14, the output is V3_OUT 1460. Using the example in FIG. 7, computer system 470 may apply MASK 1250 during forward projection (see 710) and/or backprojection (see 760). Here, MASK 1250 may be applied to identify ROI 1251 and selectively increase the resolution level of pixels or voxels associated with ROI 1251. For example, a higher resolution level within ROI 1251 may be achieved by initializing a full field-of-view (FOV) at a low resolution level for both voxels and pixels and then imposing the finer resolution level in the projection domain and iteratively solving the iterative reconstruction algorithm to fine (or refine) the resolution level in the image domain. Note that MASK 1250 is updatable during the multiresolution iterative image reconstruction.

When MASK 1250 identifies multiple ROIs 1251-1252 in the example in FIG. 14, the improved resolution level in ROIs 1251-1252 may be achieved serially or in parallel. In serial, the switch from lower to higher resolution in both the sinogram and image domains may be performed in series for each of ROIs 1251-1252. In parallel, the resolution level for all ROIs 1251-1252 may be refined simultaneously. Additional implementation details relating to multiresolution iterative reconstruction may be found in United States Patent No. US 10,517,543 entitled "Multiresolution iterative reconstruction for region of interest imaging in X-ray cone-beam computed tomography," which is incorporated herein by reference.

### Ul views

According to examples of the present disclosure, computer system 470 may generate and display UI view(s) for user 490 to interact with various sets of output volume image data. In the example in FIG. 13, a first UI view may be generated and displayed on display device 480 for user 490 to interact with V1_OUT 1210 associated with RES1. Once multiresolution iterative reconstruction is completed based on MASK 1250, a second UI view may be generated and displayed on display device 480 for user 490 to interact with multiresolution volume image data = V2_OUT 1270 that includes first voxels 1271 associated with RES2 and second voxels 1272 associated with RES3.

Similarly, in the example in FIG. 14, a first UI view may be generated and displayed on display device 480 for user 490 to interact with V1_OUT 1420 associated with RES1. Next, a second UI view may be generated and displayed on display device 480 for user 490 to interact with V2_OUT 1440 associated with RES2. Once multiresolution iterative reconstruction is completed based on MASK 1250, a third UI view may be generated and displayed on display device 480 for user 490 to interact with multiresolution volume image data = V3_OUT 1460 that includes non-ROI voxels 1461 associated with RES3 and ROI-related voxels 1462-1463 associated with RES4.

### Computer system

The above examples can be implemented by hardware (including hardware logic circuitry), software or firmware or a combination thereof. The above examples may be implemented by any suitable computing device, computer system, etc. The computer system may include processor(s), memory unit(s) and physical NIC(s) that may communicate with each other via a communication bus, etc. The computer system may include a non-transitory computer-readable medium having stored thereon instructions or program code that, when executed by the processor, cause the processor to perform processes described herein with reference to the drawings.

The techniques introduced above can be implemented in special-purpose hardwired circuitry, in software and/or firmware in conjunction with programmable circuitry, or in a combination thereof. Special-purpose hardware or hardwired circuitry may be in the form of, for example, one or more accelerators to accelerate computational tasks relating to image reconstruction, application-specific integrated circuits (ASICs), programmable logic devices (PLDs), field-programmable gate arrays (FPGAs), and others. The term "processor" is to be interpreted broadly to include a processing unit, ASIC, logic unit, or programmable gate array etc. The term "accelerator" may refer generally to any suitable hardware or other computation processing unit (e.g., high-performance computation processing unit, etc.) for accelerating computational tasks, such as graphics processing units (GPUs), tensor processing units (TPUs), neural processing units (NPUs), etc. Any alternative processor architecture(s) may be used, such as hybrid architecture (known as XPU) that is designed to handle a variety of workloads by combining different types of processing units, etc.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or any combination thereof.

Those skilled in the art will recognize that some aspects of the embodiments disclosed herein, in whole or in part, can be equivalently implemented in integrated circuits, as one or more computer programs running on one or more computers (e.g., as one or more programs running on one or more computing systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure.

Software to implement the techniques introduced here may be stored on a non-transitory computer-readable storage medium and may be executed by one or more general-purpose or special-purpose programmable microprocessors. A "computer-readable storage medium", as the term is used herein, includes any mechanism that provides (i.e., stores and/or transmits) information in a form accessible by a machine (e.g., a computer, network device, personal digital assistant (PDA), mobile device, manufacturing tool, any device with a set of one or more processors, etc.). A computer-readable storage medium may include recordable/non recordable media (e.g., read-only memory (ROM), random access memory (RAM), magnetic disk or optical storage media, flash memory devices, etc.).

The drawings are only illustrations of an example, wherein the units or procedure shown in the drawings are not necessarily essential for implementing the present disclosure. Those skilled in the art will understand that the units in the device in the examples can be arranged in the device in the examples as described or can be alternatively located in one or more devices different from that in the examples. The units in the examples described can be combined into one module or further divided into a plurality of sub-units.

### Exemplary embodiment

According to an exemplary embodiment of the present disclosure, first volume image data associated with a first resolution level may be obtained, wherein the first volume image data may be generated based on projection image data associated with a target structure of a patient. The first volume image data associated with the first resolution level may be upscaled to generate second volume image data associated with a second resolution level, wherein the second resolution level is higher than the first resolution level. Mask image data that identifies a region of interest (ROI) associated with the target structure may be obtained. Multiresolution volume image data may be generated based on the second volume image data and the mask image data, wherein the multiresolution volume image data may include at least a first voxel associated with the second resolution level outside of the ROI and a second voxel associated with a third resolution level within the ROI, the third resolution level being higher than the second resolution level. The upscaling may be along longitudinal, transverse and/or vertical axes, and the upscaling may be bilinear, cubic, and/or generated using AI. The mask image data may be generated based on the projection image data, the first volume image data, the second volume image data, segmentation data associated with the ROI, a value range associated with the ROI, a relative value difference associated with the ROI, and/or heuristics specifying one or more rules for identifying the ROI. The mask image data may be updatable during the multiresolution iterative image reconstruction and may be applied during a forward projection / backprojection operation.

### Example clauses

Further aspects of these teachings are provided by the subject matter of the following clauses (where it will be understood that any of these clauses can be combined with one or more of the other clauses as appropriate). Depending on the desired implementation, clause 2 may be combined with clause 1; clause 3 with clause 1 and/or clause 2; clause 4 with one or more of clauses 1-3; clause 5 with one or more of clauses 1-4; clause 6 with one or more of clauses 1-5, and clause 7 with one or more of clauses 1-6. This also applies to (a) clause 8, which may be combined with one or more of clauses 9-14, and (b) clause 15, which may be combined with one or more of clauses 16-21.

### (a) First aspect (iterative image reconstruction with upscaling)

Clause 1. A method for a computer system to perform iterative image reconstruction with upscaling for radiation therapy, wherein the method comprises: obtaining projection image data associated with an anatomical structure, such as a target structure of a patient; performing iterative image reconstruction for a first number of iterations to generate first volume image data based on the projection image data, wherein the first volume image data is associated with a first resolution level; upscaling the first volume image data associated with the first resolution level to generate second volume image data associated with a second resolution level, wherein the second resolution level is higher than the first resolution level; and performing iterative image reconstruction for a second number of iterations to generate output volume image data associated with the second resolution level based on the second volume image data.

Clause 2. The method of clause 1, wherein upscaling the first volume image data comprises: generating the second volume image data by upscaling the first volume image data along at least a longitudinal axis such that the second resolution level is associated with a higher longitudinal resolution compared to the first resolution level.

Clause 3. The method of clause 1 or clause 2, wherein upscaling the first volume image data comprises: performing bilinear upscaling or cubic upscaling to generate the second volume image data based on the first volume image data.

Clause 4. The method of one or more of clauses 1-3, wherein upscaling the first volume image data comprises: applying an artificial intelligence (AI) engine that is trained to perform upscaling to generate the second volume image data based on the first volume image data.

Clause 5. The method of one or more of clauses 1-4, wherein the method further comprises: upscaling the output volume image data into third volume image data associated with a third resolution level that is higher than the second resolution level; and performing iterative image reconstruction for a third number of iterations to generate final volume image data associated with the third resolution level based on the third volume image data.

Clause 6. The method of one or more of clauses 1-5, wherein performing the iterative image reconstruction comprises: performing the iterative image reconstruction to generate the first volume image data based on the projection image data, being first downsampled projection image data that is associated with the first resolution level; and performing the iterative image reconstruction to generate the output volume image data based on second downsampled projection image data that is associated with the second resolution level.

Clause 7. The method of one or more of clauses 1-6, wherein the method further comprises at least one of the following: (a) generating and displaying, on a display device, a first user interface (UI) view to allow a user to interact with the first volume image data, wherein the first UI view specifies at least one of the following: the first resolution level and the first number of iterations; and (b) generating and displaying, on the display device, a second UI view to allow the user to interact with the output volume image data, wherein the second UI view specifies at least one of the following: the second resolution level, and a total number of iterations that includes the first number of iterations and the second number of iterations.

Clause 8. A non-transitory computer-readable medium having stored thereon instructions that, when executed by a processor, cause the processor to perform the following: obtain projection image data associated with an anatomical structure, such as a target structure of a patient; perform iterative image reconstruction for a first number of iterations to generate first volume image data based on the projection image data, wherein the first volume image data is associated with a first resolution level; upscale the first volume image data associated with the first resolution level to generate second volume image data associated with a second resolution level, wherein the second resolution level is higher than the first resolution level; and perform iterative image reconstruction for a second number of iterations to generate output volume image data associated with the second resolution level based on the second volume image data.

Clause 9. The non-transitory computer-readable medium of clause 8, wherein the instructions for upscaling the first volume image data cause the processor to: generate the second volume image data by upscaling the first volume image data along at least a longitudinal axis such that the second resolution level is associated with a higher longitudinal resolution compared to the first resolution level.

Clause 10. The non-transitory computer-readable medium of clause 8 or clause 9, wherein the instructions for upscaling the first volume image data cause the processor to: perform bilinear upscaling or cubic upscaling to generate the second volume image data based on the first volume image data.

Clause 11. The non-transitory computer-readable medium of one or more of clauses 8-10, wherein the instructions for upscaling the first volume image data cause the processor to: apply an AI engine that is trained to perform upscaling to generate the second volume image data based on the first volume image data.

Clause 12. The non-transitory computer-readable medium of one or more of clauses 8-11, wherein the instructions further cause the processor to: upscale the output volume image data into third volume image data associated with a third resolution level that is higher than the second resolution level; and perform iterative image reconstruction for a third number of iterations to generate final volume image data associated with the third resolution level based on the third volume image data.

Clause 13. The non-transitory computer-readable medium of one or more of clauses 8-12, wherein the instructions for performing the iterative image reconstruction cause the processor to: perform the iterative image reconstruction to generate the first volume image data based on the projection image data, being first downsampled projection image data that is associated with the first resolution level; and perform the iterative image reconstruction to generate the output volume image data based on second downsampled projection image data that is associated with the second resolution level.

Clause 14. The non-transitory computer-readable medium of one or more of clauses 8-13, wherein the instructions further cause the processor to perform at least one of the following: (a) generate and display, on a display device, a first user interface (UI) view to allow a user to interact with the first volume image data, wherein the first UI view specifies at least one of the following: the first resolution level and the first number of iterations; and (b) generate and display, on the display device, a second UI view to allow the user to interact with the output volume image data, wherein the second UI view specifies at least one of the following: the second resolution level, and a total number of iterations that includes the first number of iterations and the second number of iterations. Depending on the desired implementation, there is further provided a computer system comprising a processor and a non-transitory computer-readable medium according to one or more of clauses 8-14.

Clause 15. An imaging system, comprising: an imaging source and a detector to acquire projection image data associated with an anatomical structure, such as a target structure of a patient; and a computer system configured to: perform iterative image reconstruction for a first number of iterations to generate first volume image data based on the projection image data, wherein the first volume image data is associated with a first resolution level; upscale the first volume image data associated with the first resolution level to generate second volume image data associated with a second resolution level, wherein the second resolution level is higher than the first resolution level; and perform iterative image reconstruction for a second number of iterations to generate output volume image data associated with the second resolution level based on the second volume image data.

Clause 16. The imaging system of clause 15, wherein the computer system is configured to upscale the first volume image data by: generating the second volume image data by upscaling the first volume image data along at least a longitudinal axis such that the second resolution level is associated with a higher longitudinal resolution compared to the first resolution level.

Clause 17. The imaging system of clause 15 or clause 16, wherein the computer system is configured to upscale the first volume image data by: performing bilinear upscaling or cubic upscaling to generate the second volume image data based on the first volume image data.

Clause 18. The imaging system of one or more of clauses 15-17, wherein the computer system is configured to upscale the first volume image data by: applying an artificial intelligence (AI) engine that is trained to perform upscaling to generate the second volume image data based on the first volume image data.

Clause 19. The imaging system of one or more of clauses 15-18, wherein the computer system is further configured to: upscale the output volume image data into third volume image data associated with a third resolution level that is higher than the second resolution level; and perform iterative image reconstruction for a third number of iterations to generate final volume image data associated with the third resolution level based on the third volume image data.

Clause 20. The imaging system of one or more of clauses 15-19, wherein the computer system is configured to perform the iterative image reconstruction by: generating the first volume image data based on the projection image data, being first downsampled projection image data that is associated with the first resolution level; and generating the output volume image data based on second downsampled projection image data that is associated with the second resolution level.

Clause 21. The imaging system of one or more of clauses 15-20, wherein the computer system is further configured to perform at least one of the following: (a) generate and display, on a display device, a first user interface (UI) view to allow a user to interact with the first volume image data, wherein the first UI view specifies at least one of the following: the first resolution level and the first number of iterations; and (b) generate and display, on the display device, a second UI view to allow the user to interact with the output volume image data, wherein the second UI view specifies at least one of the following: the second resolution level, and a total number of iterations that includes the first number of iterations and the second number of iterations.

Clause 22. A computer program comprising instructions which, when the computer program is executed by a computer, cause the computer to perform the following steps: obtain projection image data associated with an anatomical structure such as a target structure ; perform iterative image reconstruction for a first number of iterations to generate first volume image data based on the projection image data, wherein the first volume image data is associated with a first resolution level; upscale the first volume image data associated with the first resolution level to generate second volume image data associated with a second resolution level, wherein the second resolution level is higher than the first resolution level; and perform iterative image reconstruction for a second number of iterations to generate output volume image data associated with the second resolution level based on the second volume image data.

Clause 23. The computer program of clause 22, wherein the instructions for upscaling the first volume image data cause the computer to: generate the second volume image data by upscaling the first volume image data along at least a longitudinal axis such that the second resolution level is associated with a higher longitudinal resolution compared to the first resolution level.

Clause 24. The computer program of clause 22 or clause 23, wherein the instructions for upscaling the first volume image data cause the computer to: perform bilinear upscaling or cubic upscaling to generate the second volume image data based on the first volume image data.

Clause 25. The computer program of one or more of clauses 21-24, wherein the instructions for upscaling the first volume image data cause the computer to: apply an AI engine that is trained to perform upscaling to generate the second volume image data based on the first volume image data.

Clause 26. The computer program of one or more of clauses 21-25, wherein the instructions further cause the computer to: upscale the output volume image data into third volume image data associated with a third resolution level that is higher than the second resolution level; and perform iterative image reconstruction for a third number of iterations to generate final volume image data associated with the third resolution level based on the third volume image data.

Clause 27. The computer program of one or more of clauses 21-26, wherein the instructions for performing the iterative image reconstruction cause the computer to: perform the iterative image reconstruction to generate the first volume image data based on the projection image data, being first downsampled projection image data that is associated with the first resolution level; and perform the iterative image reconstruction to generate the output volume image data based on second downsampled projection image data that is associated with the second resolution level.

Clause 28. The computer program of one or more of clauses 21-27, wherein the instructions further cause the computer to perform at least one of the following: (a) generate and display, on a display device, a first user interface (UI) view to allow a user to interact with the first volume image data, wherein the first UI view specifies at least one of the following: the first resolution level and the first number of iterations; and (b) generate and display, on the display device, a second UI view to allow the user to interact with the output volume image data, wherein the second UI view specifies at least one of the following: the second resolution level, and a total number of iterations that includes the first number of iterations and the second number of iterations.

### (b) Second aspect (multiresolution image reconstruction with upscaling)

Clause 1. A method for a computer system to perform multiresolution image reconstruction with upscaling, wherein the method comprises: obtaining first volume image data associated with a first resolution level, wherein the first volume image data is generated based on projection image data associated with an anatomical structure, such as a target structure of a patient; upscaling the first volume image data associated with the first resolution level to generate second volume image data associated with a second resolution level, wherein the second resolution level is higher than the first resolution level; obtaining mask image data that identifies a region of interest (ROI) associated with the anatomical or target structure; and generating multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

Clause 2. The method of clause 1, wherein upscaling the first volume image data comprises: generating the second volume image data by upscaling the first volume image data along at least a longitudinal axis such that the second resolution level is associated with a higher longitudinal resolution compared to the first resolution level.

Clause 3. The method of clause 1 or clause 2, wherein upscaling the first volume image data comprises at least one of the following: performing bilinear upscaling or cubic upscaling to generate the second volume image data based on the first volume image data; and applying an artificial intelligence (AI) engine that is trained to perform upscaling to generate the second volume image data based on the first volume image data.

Clause 4. The method of one or more of clauses 1-3, wherein obtaining the first volume image data comprises: performing iterative image reconstruction to generate the first volume image data based on the projection image data.

Clause 5. The method of one or more of clauses 1-4, wherein obtaining the mask image data comprises: obtaining the mask image data that is generated based on one or more of the following input data: the projection image data, the first volume image data, the second volume image data and segmentation data associated with the ROI.

Clause 6. The method of one or more of clauses 1-5, wherein obtaining the mask image data comprises: obtaining the mask image data that is generated based on one or more of the following: a value range associated with the ROI, a relative value difference associated with the ROI, and heuristics specifying one or more rules for identifying the ROI.

Clause 7. The method of one or more of clauses 1-6, wherein generating the multiresolution volume image data comprises: performing multiresolution iterative image reconstruction based on the second volume image data and the mask image data, wherein the mask image data is updatable during the multiresolution iterative image reconstruction and applied during at least one of the following: a forward projection operation and a backprojection operation.

Clause 8. A non-transitory computer-readable medium having stored thereon instructions that, when executed by a processor, cause the processor to perform the following: obtain first volume image data associated with a first resolution level, wherein the first volume image data is generated based on projection image data associated with an anatomical structure, such as a target structure of a patient; upscale the first volume image data associated with the first resolution level to generate second volume image data associated with a second resolution level, wherein the second resolution level is higher than the first resolution level; obtain mask image data that identifies a region of interest (ROI) associated with the anatomical or target structure; and generate multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

Clause 9. The non-transitory computer-readable medium of clause 8, wherein the instructions for upscaling the first volume image data cause the processor to: generate the second volume image data by upscaling the first volume image data along at least a longitudinal axis such that the second resolution level is associated with a higher longitudinal resolution compared to the first resolution level.

Clause 10. The non-transitory computer-readable medium of clause 8 or clause 9, wherein the instructions for upscaling the first volume image data cause the processor to perform at least one of the following: perform bilinear upscaling or cubic upscaling to generate the second volume image data based on the first volume image data; and apply an artificial intelligence (AI) engine that is trained to perform upscaling to generate the second volume image data based on the first volume image data.

Clause 11. The non-transitory computer-readable medium of one or more of clauses 8-10, wherein the instructions for obtaining the first volume image data comprises: performing iterative image reconstruction to generate the first volume image data based on the projection image data.

Clause 12. The non-transitory computer-readable medium of one or more of clauses 8-11, wherein the instructions for obtaining the mask image data cause the processor to: obtain the mask image data that is generated based on one or more of the following input data: the projection image data, the first volume image data, the second volume image data and segmentation data associated with the ROI.

Clause 13. The non-transitory computer-readable medium of one or more of clauses 8-12, wherein the instructions for obtaining the mask image data cause the processor to: obtain the mask image data that is generated based on one or more of the following: a value range associated with the ROI, a relative value difference associated with the ROI, and heuristics specifying one or more rules for identifying the ROI.

Clause 14. The non-transitory computer-readable medium of one or more of clauses 8-13, wherein the instructions for generating the multiresolution volume image data cause the processor to: perform multiresolution iterative image reconstruction based on the second volume image data and the mask image data, wherein the mask image data is updatable during the multiresolution iterative image reconstruction and applied during at least one of the following: a forward projection operation and a backprojection operation. Depending on the desired implementation, there is further provided a computer system comprising a processor and a non-transitory computer-readable medium according to one or more of clauses 8-14.

Clause 15. An imaging system, comprising: an imaging source and a detector to acquire projection image data associated with an anatomical structure, such as a target structure of a patient; and a computer system configured to: obtain first volume image data associated with a first resolution level, wherein the first volume image data is generated based on the projection image data; upscale the first volume image data associated with the first resolution level to generate second volume image data associated with a second resolution level, wherein the second resolution level is higher than the first resolution level; obtain mask image data that identifies a region of interest (ROI) associated with the anatomical or target structure; and generate multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

Clause 16. The imaging system of clause 15, wherein the computer system is configured to upscale the first volume image data by: generating the second volume image data by upscaling the first volume image data along at least a longitudinal axis such that the second resolution level is associated with a higher longitudinal resolution compared to the first resolution level.

Clause 17. The imaging system of clause 15 or clause 16, wherein the computer system is configured to upscale the first volume image data by performing at least one of the following: performing bilinear upscaling or cubic upscaling to generate the second volume image data based on the first volume image data; and applying an artificial intelligence (AI) engine that is trained to perform upscaling to generate the second volume image data based on the first volume image data.

Clause 18. The imaging system of one or more of clauses 15-17, wherein the computer system is configured to obtain the first volume image data by: performing iterative image reconstruction to generate the first volume image data based on the projection image data.

Clause 19. The imaging system of one or more of clauses 15-18, wherein the computer system is configured to obtain the mask image data by: obtaining the mask image data that is generated based on one or more of the following input data: the projection image data, the first volume image data, the second volume image data and segmentation data associated with the ROI.

Clause 20. The imaging system of one or more of clauses 15-19, wherein the computer system is configured to obtain the mask image data by: obtaining the mask image data that is generated based on one or more of the following: a value range associated with the ROI, a relative value difference associated with the ROI, and heuristics specifying one or more rules for identifying the ROI.

Clause 21. The imaging system of one or more of clauses 15-20, wherein the computer system is configured to generate the multiresolution volume image data by: performing multiresolution iterative image reconstruction based on the second volume image data and the mask image data, wherein the mask image data is updatable during the multiresolution iterative image reconstruction and applied during at least one of the following: a forward projection operation and a backprojection operation.

Clause 22. A computer program comprising instructions which, when the computer program is executed by a computer, cause the computer to perform the following steps: obtain first volume image data associated with a first resolution level, wherein the first volume image data is generated based on projection image data associated with an anatomical structure, such as a target structure of a patient; upscale the first volume image data associated with the first resolution level to generate second volume image data associated with a second resolution level, wherein the second resolution level is higher than the first resolution level; obtain mask image data that identifies a region of interest (ROI) associated with the anatomical or target structure; and generate multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

Clause 23. The computer program of clause 22, wherein the instructions for upscaling the first volume image data cause the computer to: generate the second volume image data by upscaling the first volume image data along at least a longitudinal axis such that the second resolution level is associated with a higher longitudinal resolution compared to the first resolution level.

Clause 24. The computer program of clause 22 or clause 23, wherein the instructions for upscaling the first volume image data cause the computer to perform at least one of the following: perform bilinear upscaling or cubic upscaling to generate the second volume image data based on the first volume image data; and apply an artificial intelligence (AI) engine that is trained to perform upscaling to generate the second volume image data based on the first volume image data.

Clause 25. The computer program of one or more of clauses 22-24, wherein the instructions for obtaining the first volume image data cause the computer to: perform iterative image reconstruction to generate the first volume image data based on the projection image data.

Clause 26. The computer program of one or more of clauses 22-25, wherein the instructions for obtaining the mask image data cause the computer to: obtain the mask image data that is generated based on one or more of the following input data: the projection image data, the first volume image data, the second volume image data and segmentation data associated with the ROI.

Clause 27. The computer program of one or more of clauses 22-26, wherein the instructions for obtaining the mask image data cause the computer to: obtain the mask image data that is generated based on one or more of the following: a value range associated with the ROI, a relative value difference associated with the ROI, and heuristics specifying one or more rules for identifying the ROI.

Clause 28. The computer program of one or more of clauses 22-27, wherein the instructions for generating the multiresolution volume image data cause the computer to: perform multiresolution iterative image reconstruction based on the second volume image data and the mask image data, wherein the mask image data is updatable during the multiresolution iterative image reconstruction and applied during at least one of the following: a forward projection operation and a backprojection operation.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method for a computer system to perform multiresolution image reconstruction with upscaling, wherein the method comprises:
obtaining first volume image data associated with a first resolution level, wherein the first volume image data is generated based on projection image data associated with a target structure of a patient;
upscaling the first volume image data associated with the first resolution level to generate second volume image data associated with a second resolution level, wherein the second resolution level is higher than the first resolution level;
obtaining mask image data that identifies a region of interest (ROI) associated with the target structure; and
generating multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

2. The method of claim 1, wherein upscaling the first volume image data comprises:
generating the second volume image data by upscaling the first volume image data along at least a longitudinal axis such that the second resolution level is associated with a higher longitudinal resolution compared to the first resolution level.

3. The method of claim 1 or claim 2, wherein upscaling the first volume image data comprises at least one of the following:
performing bilinear upscaling or cubic upscaling to generate the second volume image data based on the first volume image data; and
applying an artificial intelligence (AI) engine that is trained to perform upscaling to generate the second volume image data based on the first volume image data.

4. The method of any preceding claim, wherein obtaining the first volume image data comprises:
performing iterative image reconstruction to generate the first volume image data based on the projection image data.

5. The method of any preceding claim, wherein obtaining the mask image data comprises:
obtaining the mask image data that is generated based on one or more of the following input data: the projection image data, the first volume image data, the second volume image data and segmentation data associated with the ROI.

6. The method of any preceding claim, wherein obtaining the mask image data comprises:
obtaining the mask image data that is generated based on one or more of the following: a value range associated with the ROI, a relative value difference associated with the ROI, and heuristics specifying one or more rules for identifying the ROI.

7. The method of any preceding claim, wherein generating the multiresolution volume image data comprises:
performing multiresolution iterative image reconstruction based on the second volume image data and the mask image data, wherein the mask image data is updatable during the multiresolution iterative image reconstruction and applied during at least one of the following: a forward projection operation and a backprojection operation.

8. A non-transitory computer-readable medium having stored thereon instructions that, when executed by a processor, cause the processor to perform the following:
obtain first volume image data associated with a first resolution level, wherein the first volume image data is generated based on projection image data associated with a target structure of a patient;
upscale the first volume image data associated with the first resolution level to generate second volume image data associated with a second resolution level, wherein the second resolution level is higher than the first resolution level;
obtain mask image data that identifies a region of interest (ROI) associated with the target structure; and
generate multiresolution volume image data based on the second volume image data and the mask image data, wherein the multiresolution volume image data includes at least a first voxel associated with the second resolution level outside of the ROI and a second voxel associated with a third resolution level within the ROI, the third resolution level being higher than the second resolution level.

9. The non-transitory computer-readable medium of claim 8, wherein the instructions for upscaling the first volume image data cause the processor to:
generate the second volume image data by upscaling the first volume image data along at least a longitudinal axis such that the second resolution level is associated with a higher longitudinal resolution compared to the first resolution level.

10. The non-transitory computer-readable medium of claim 8 or claim 9, wherein the instructions for upscaling the first volume image data cause the processor to:
perform bilinear upscaling or cubic upscaling to generate the second volume image data based on the first volume image data; and
apply an artificial intelligence (AI) engine that is trained to perform upscaling to generate the second volume image data based on the first volume image data.

11. The non-transitory computer-readable medium of any of claim 8 to claim 10, wherein the instructions for obtaining the first volume image data cause the processor to:
perform iterative image reconstruction to generate the first volume image data based on the projection image data.

12. The non-transitory computer-readable medium of any of claim 8 to claim 11, wherein the instructions for obtaining the mask image data cause the processor to:
obtain the mask image data that is generated based on one or more of the following input data: the projection image data, the first volume image data, the second volume image data and segmentation data associated with the ROI.

13. The non-transitory computer-readable medium of any of claim 8 to claim 12, wherein the instructions for obtaining the mask image data cause the processor to:
obtain the mask image data that is generated based on one or more of the following: a value range associated with the ROI, a relative value difference associated with the ROI, and heuristics specifying one or more rules for identifying the ROI.

14. The non-transitory computer-readable medium of any of claim 8 to claim 13, wherein the instructions for generating the multiresolution volume image data cause the processor to:
perform multiresolution iterative image reconstruction based on the second volume image data and the mask image data, wherein the mask image data is updatable during the multiresolution iterative image reconstruction and applied during at least one of the following: a forward projection operation and a backprojection operation.

15. An imaging system, comprising:
an imaging source and a detector to acquire projection image data associated with a target structure of a patient; and
a computer system configured to:
obtain first volume image data associated with a first resolution level, wherein the first volume image data is generated based on the projection image data associated with the target structure;
upscale the first volume image data associated with the first resolution level to generate second volume image data associated with a second resolution level, wherein the second resolution level is higher than the first resolution level;
obtain mask image data that identifies a region of interest (ROI) associated with the target structure; and
generate multiresolution volume image data based on the second volume image data and the mask image data, wherein the multiresolution volume image data includes at least a first voxel associated with the second resolution level outside of the ROI and a second voxel associated with a third resolution level within
the ROI, the third resolution level being higher than the second resolution level;
and optionally:
wherein the computer system is further configured to perform the method as defined in any of claim 2 to claim 7.
